# EUROPEAN PATENT SPECIFICATION

(11) **EP 4 751 667 B1**
(45) Date of publication and mention of the grant of the patent: **30.09.2026**
(21) Application number: 24216938.1
(22) Date of filing: 02.12.2024
(51) Int. Cl.: A61B 17/70

(54) **INSTRUMENT FOR USE WITH A BONE ANCHORING DEVICE**
INSTRUMENT ZUR VERWENDUNG MIT EINER KNOCHENVERANKERUNGSVORRICHTUNG
INSTRUMENT DESTINÉ À ÊTRE UTILISÉ AVEC UN DISPOSITIF D'ANCRAGE OSSEUX

(43) Date of publication of application: 03.06.2026
(73) Proprietor: Biedermann Technologies GmbH & Co. KG, 78166 Donaueschingen (DE)
(72) Inventor: BIEDERMANN, Timo, 78647 Trossingen (DE); SCHÜNEMANN, Achim, 78054 Villingen-Schwenningen (DE)
(74) Representative: Prüfer & Partner mbB Patentanwälte · Rechtsanwälte

(56) References cited:
- US-A1- 2021 393 299
- US-A1- 2022 160 401
- US-A1- 2022 330 989
- US-B2- 12 042 188

## Description

The invention relates to an instrument for use with a bone anchoring device and to a system of such an instrument and a bone anchoring device. In particular, the instrument may be an instrument for locking and unlocking a head of a bone anchor in a receiving part of the bone anchoring device, and/or the instrument may be an instrument for positioning and re-positioning a receiving part of the bone anchoring device relative to the bone anchoring element.

In spinal surgery, often multiple segments of the spinal column have to be corrected and/or stabilized using a spinal rod and polyaxial bone anchors. During such a procedure, repeated adjustments of the bone anchoring elements and the rod relative to the receiving parts of a respective polyaxial bone anchoring devices may become necessary.

Usually, a polyaxial bone anchoring device includes a coupling device and a bone anchoring element with a head that is pivotably received in the coupling device and can be locked at a desired angle of the bone anchoring element relative to the coupling device. The coupling device also receives a rod that is configured to connect the polyaxial bone anchoring device to a further bone anchor.

US 2022/0160401 A1 describes an instrument for placing a coupling device onto a head of a bone anchor, the coupling device comprising a receiving part comprising a head receiving portion for pivotably receiving the head of the bone anchor and a rod receiving portion for receiving a rod, and a locking ring movable around the head receiving portion from an insertion position where the head of the bone anchor is insertable into the head receiving portion to a pre-locking position where the head is prevented from removal from the head receiving portion. The instrument has a longitudinal axis and comprises a holding member having a front portion engageable with the receiving part and a pushing member that is movable axially relative to the holding member to engage the locking ring.

US 12,042,188 B2 describes a surgical instrument comprising a member being engageable to a spinal implant configured for connection to a bone fastener shaft. An actuator is connected to the member. A latch is connected to the actuator and the connection is configured to change between at least one non-locked orientation such that the actuator is movable relative to the member and a locked orientation such that the actuator is fixed relative to the member.

US 10,470,805 B2 describes an instrument suitable for performing such repeated adjustments of the bone anchoring elements relative to a rod. The instruments includes a tube assembly comprising an inner tube, an outer tube, the tube assembly having a longitudinal axis, wherein the outer tube is displaceable relative to the inner tube from a first axial position to a second axial position and vice versa, and wherein the first axial position is associated with an unlocking configuration of a polyaxial bone anchoring device in which a head of a bone anchor is pivotable, and the second axial position is associated with a locking configuration of the polyaxial bone anchoring device in which the head is locked. The instrument further comprises an actuator assembly with an actuating mechanism for displacing the inner tube, relative to the outer tube, wherein the actuator assembly is rotatable around the longitudinal axis to a first rotational position wherein the actuator assembly actuates the tube assembly to assume the first axial position and to a second rotational position wherein the actuator assembly actuates the tube assembly to assume the second axial position.

There may be a need for further simplification of the handling of such an instrument and/or for a greater variety of applications.

It is an object of the invention to provide an improved instrument for carrying out revisions or further positioning or repositioning of a polyaxial bone anchoring device with respect to a rod during surgery that is simple to handle for a user and that permits a greater variety of correction steps during surgery. It is a further object to provide a system of such an instrument and a polyaxial bone anchoring device adapted for use with the instrument.

The object is solved by in instrument according to claim 1 and by a system according to claim 15. Further developments are given in the dependent claims.

According to an embodiment, the instrument is adapted for use with a bone anchoring device that comprises a bone anchoring element and a receiving part for coupling a rod to the bone anchoring element, and the instrument comprises at least one tube attachable to the receiving part of the bone anchoring device, the tube defining a longitudinal axis, and at least one alignment member that is configured to assist in attaching the tube to the receiving part .

According to a further embodiment, an instrument for locking and unlocking a head of a bone anchor in a receiving part of a bone anchoring device includes a tube assembly attachable to the bone anchoring device, the tube assembly comprising at least a first tube and a second tube defining a longitudinal axis, the first and second tubes being configured to engage the bone anchoring device and being movable relative to one another between a first axial position associated with an unlocking configuration in which the head is unlocked in the receiving part, and a second axial position associated with a locking configuration in which the head is locked in the receiving part, and at least one alignment member that is configured to assist in attaching the tube assembly to the bone anchoring device, wherein the instrument further comprises an actuator assembly comprising an actuating mechanism configured to move the first and second tubes of the tube assembly from the first axial position to the second axial position, and vice versa.

The alignment member assists in attaching and/or detaching the tube or tube assembly to the bone anchoring device by guiding the tube or tube assembly relative to the receiving part of the bone anchoring device.

**In** an embodiment, the alignment member may be an outer alignment member, such as an outer sleeve, that at least partially encloses the tube, or first and second tubes, in a circumferential direction thereof. The outer alignment member may indicate a rotational position of the tube or the tube assembly in which the tube or tube assembly engages the receiving part so as to be coupled to the receiving part, or in which the tube or tube assembly is disengaged from the receiving part so as to be mountable to and removable from the receiving part. Thus, mounting of the tube or tube assembly onto the receiving part of the bone anchoring device may be facilitated. Alternatively, or in addition, the alignment member may be an inner alignment member, such as a plunger, arranged at least partially within the tube or within the first and second tubes of the tube assembly. Preferably, the inner alignment member is configured to press onto a rod inserted into the receiving part. This may facilitate mounting of the tube or tube assembly onto the receiving part, in particular by bringing or holding the rod in a lowermost position within the receiving part and/or providing for additional guidance when placing the tube assembly onto the receiving part.

According to another embodiment an instrument for locking and unlocking a head of a bone anchor in a receiving part of a bone anchoring device includes a tube assembly attachable to the bone anchoring device, the tube assembly comprising at least a first tube and a second tube defining a longitudinal axis, the first and second tubes being configured to engage the bone anchoring device and being movable relative to one another between a first axial position associated with an unlocking configuration in which the head is unlocked in the receiving part, and a second axial position associated with a locking configuration in which the head is locked in the receiving part, wherein the instrument further comprises an actuator assembly comprising an actuating mechanism configured to move the first and second tubes of the tube assembly from the first axial position to the second axial position, and vice versa, and wherein the actuator assembly is configured to assume at least a third configuration in which the actuator assembly is connectable to and/or removable from the tube assembly, and a securing mechanism preventing the actuator assembly from assuming the third configuration. Preferably, the securing mechanism is configured such that it can be released by unscrewing a handle portion of the actuator assembly.

The securing mechanism can improve handling of the instrument and improve its safety during surgery, in particular by preventing unintended release of the actuator assembly from the tube assembly.

The actuator assembly may be removably connectable to the tube assembly, thereby it is possible to adjust a plurality of bone anchoring devices, in particular polyaxial bone anchoring devices, that each are connected to a tube assembly with one single actuator assembly one after the other. As the tube assemblies may be positioned close to each other, operating with one single actuator assembly is convenient in view of the reduced available space. The tube assemblies may be all the same or differ with respect to their length or other features such as the engagement features for engagement with the (polyaxial) bone anchoring device. Hence, the instrument may also provide a modular system that includes an actuator assembly and different tube assemblies that are adapted to be used with the actuator assembly. Moreover, with the instrument according to embodiments, a sensor member and/or a navigation member configured to detect at least one of the unlocking configuration and the locking configuration of the head in the receiving part or the first axial position and the second axial position of the first and second tubes is provided. The instrument or sensor member may additionally produce a signal, in particular an optical and/or audible signal, when it enters one of the different configurations of locking, unlocking, or removing. This further increases the safety and user-friendliness.

The sensor member and/or navigation member may comprise a base member connectable to the instrument and a movable element, such as a sensor element or a pin, that is movable relative to the base member. The sensor member and/or navigation member may be configured such that a movement of the first and second tubes of the tube assembly relative to one another between the first axial position and the second axial position, or vice versa, causes a movement of the movable element relative to the base member. The movement of the movable element may be detected by a sensor device, wherein the sensor device may in particular be configured to measure a distance of the sensor element relative to a reference position, and/or a displacement of the sensor element, and/or a pressure acting on the sensor element and/or can be a position sensor configured to detect the position of the sensor element. Alternatively or in addition, the movable element may be provided with a navigation sphere, and the navigation member may be configured to detect a relative or absolute displacement of the navigation sphere.

**In** addition, with the instrument the correction steps that are necessary for adjusting the bone anchoring elements and the rod and also the adjustment of the position of one or more vertebrae are simplified. A temporary locking of the bone anchoring element in the receiving part can be effected using only the instrument with the rod already inserted into the receiving part but without the aid of a locking element such as a set screw. As a result thereof, the polyaxial bone anchoring device allows to adjust or re-adjust an angular position of the receiving part relative to the bone anchoring device several times while the rod is already inserted. The temporary locking of the head using the instrument also permits to use the polyaxial bone anchoring device like a monoaxial bone anchoring device.

Further features and advantages of the invention will become apparent from the description of embodiments by means of the accompanying drawings. **In** the drawings:
- Fig. 1: shows a perspective view of the instrument and a bone anchoring device according to an embodiment.
- Fig. 2: shows an exploded perspective view of a tube assembly of the instrument of Fig. 1.
- Fig. 3: shows a perspective view of the tube assembly of Fig. 2 in an assembled state.
- Fig. 4: shows a perspective view of an outer tube of the tube assembly of Figs. 2 and 3.
- Fig. 5: shows a perspective view of an inner tube of the tube assembly of Figs. 2 and 3.
- Fig. 6a: shows an enlarged perspective view of an upper portion of the tube assembly of Fig. 3 in a first configuration.
- Fig. 6b: shows an enlarged perspective view of an upper portion of the tube assembly of Fig. 3 in a second configuration.
- Fig. 7: shows an exploded perspective view of a plunger of the tube assembly of Figs. 2 and 3.
- Fig. 8: shows a perspective view of the plunger of Fig. 7 in an assembled state.
- Fig. 9: shows a first perspective view of an outer sleeve of the tube assembly of Figs. 2 and 3.
- Fig. 10: shows a second perspective view of the outer sleeve of the tube assembly of Figs. 2, 3 and 9.
- Fig. 11: shows a cross-sectional view of portions of the tube assembly of Fig. 3, wherein the cross-section is taken in a plane that includes a longitudinal axis of the tube assembly.
- Fig. 12: shows enlarged cross-sectional views of an upper portion and of a lower portion of the tube assembly of Fig. 11.
- Fig. 13: shows an exploded perspective view of an actuator assembly of the instrument of Fig. 1.
- Fig. 14: shows a perspective view of the actuator assembly of Fig. 13 in an assembled state.
- Fig. 15: shows an enlarged view of a portion of the actuator assembly of Fig. 14.
- Fig. 16: shows a perspective view of a second inner tube of the actuator assembly of Figs. 13 and 14.
- Fig. 17: shows a perspective view of a first inner tube of the actuator assembly of Figs. 13 and 14.
- Fig. 18a: shows a perspective view of an adjustment member of the actuator assembly of Figs. 13 and 14 when viewed at an angle from slightly above.
- Fig. 18b: shows a perspective view of the adjustment member of Fig. 18a when viewed at an angle from slightly below.
- Fig. 19: shows a top view of the adjustment member of Figs. 18a and 18b.
- Fig. 20: shows an exploded perspective view of the bone anchoring device of Fig. 1.
- Fig. 21: shows a perspective view of the bone anchoring device of Fig. 20 in an assembled state.
- Fig. 22: shows an enlarged perspective view of a portion of the bone anchoring device of Fig. 21.
- Figs. 23a to 23c: show perspective views of steps of connecting the instrument of Fig. 1 to the bone anchoring device of Fig. 21.
- Figs. 24a to 24c: show enlarged views of a lower portion of the instrument of Fig. 1 and of an upper portion of the bone anchoring device of Fig. 21 during the steps of Figs. 23a to 23c.
- Figs. 25a and 25b: show cross-sectional views of a lower portion of the instrument of Fig. 1 and of an upper portion of the bone anchoring device of Fig. 21 during locking and unlocking an inserted head in the bone anchoring device, wherein the cross-section is taken in a plane extending through the central axis of the receiving part and perpendicular to an axis of an inserted rod.
- Fig. 26: shows a perspective view of the instrument according to a further embodiment.
- Fig. 27: shows a perspective view of the instrument of Fig. 26 in an assembled state of a sensor member of the instrument.
- Fig. 28: shows an enlarged perspective view of a portion of instrument of Fig. 26.
- Fig. 29: shows an enlarged perspective view of a portion of instrument of Figs. 26 to 28 in the assembled state of the sensor member.
- Fig. 30: shows a perspective view onto an outer surface of the sensor member of the instrument of Figs. 26 to 29, wherein the sensor member is viewed slightly from above.
- Fig. 31: shows a perspective view onto an inner surface of the sensor member of Fig. 30, wherein the sensor member is viewed slightly from above.
- Fig. 32: shows a top view of the sensor member of Fig. 30.
- Fig. 33: shows a side view onto the outer surface of the sensor member of Fig. 30.
- Fig. 34: shows a side view onto the inner surface of the sensor member of Fig. 30.
- Figs. 35a and 35b: show cross-sectional views of a portion of the instrument of Fig. Fig. 26 in an assembled state of the sensor member during locking and unlocking an inserted head in the bone anchoring device, wherein the cross-section is taken in a plane extending through the longitudinal axis of the tube assembly.
- Figs. 36a and 36b: show perspective views onto the inner surface of the sensor member of Fig. 30 during locking and unlocking of the head.
- Fig. 37: shows a perspective view of the instrument according to a further embodiment.
- Fig. 38: shows a perspective view of a navigation member of the instrument of Fig. 37 when viewed from slightly below.
- Fig. 39: shows a perspective side view of the navigation member of Fig. 38.
- Fig. 40: shows a perspective rear view of the navigation member of Fig. 38.
- Fig. 41: shows an enlarged perspective view of a portion of the navigation member of Fig. 40.
- Fig. 42: shows a perspective view of a spinal column with a plurality of polyaxial bone anchoring devices and with an instrument of Fig. 1 connected to a first one of the polyaxial bone anchoring devices and an instrument according to a further embodiment connected to a second one of the polyaxial bone anchoring devices.
- Fig. 43: shows a perspective view of the instrument according to the further embodiment of Fig. 42 in an assembled state of the instrument.
- Fig. 44: shows a perspective view of a tube of the instrument of Fig. 43.
- Fig. 45: shows a perspective view of an outer sleeve of the instrument of Fig. 43.

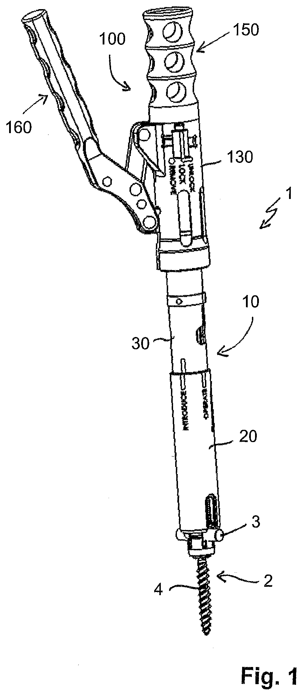

Fig. 1 shows an embodiment of an instrument 1 for use with a polyaxial bone anchoring device 2, the polyaxial bone anchoring device 2 being configured for coupling a rod 3 to a bone anchoring element 4. The instrument 1 comprises a tube assembly 10 and an actuator assembly 100 that is removably connectable to the tube assembly 10. As shown in Fig. 1, the tube assembly 10 is attachable to the polyaxial bone anchoring device 2.

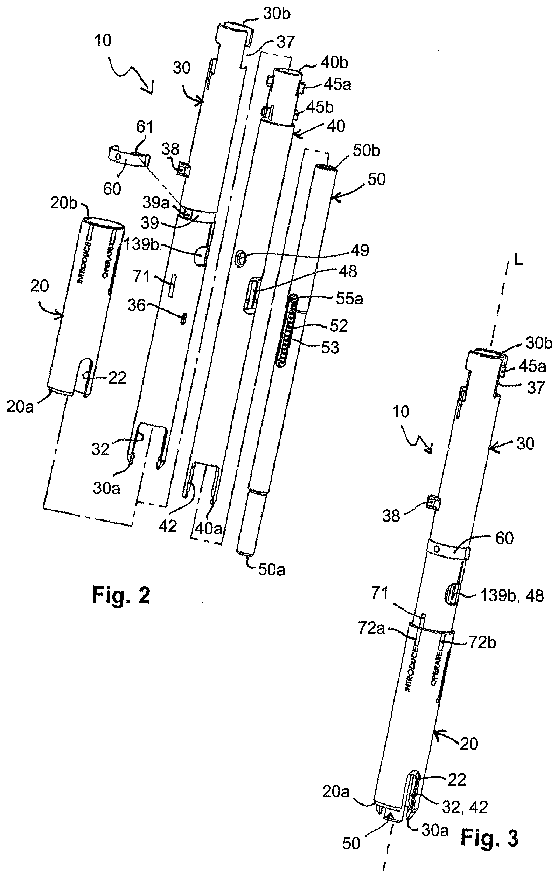

The tube assembly 10 will be described with additional reference to Figs. 2 to 12. The tube assembly 10 comprises an outer sleeve 20, an outer tube 30, an inner tube 40 and a plunger 50. The inner tube 40 may be a first tube and the outer tube 30 may be a second tube. The outer tube 30 and the inner tube 40 define a longitudinal axis or tube axis L. In an assembled state of the tube assembly 10, the outer sleeve 20 entirely encloses a lower portion of the outer tube 30 in a circumferential direction thereof, the inner tube 40 is arranged within the outer tube 30, and the plunger 50 is arranged within the inner tube 40. The outer sleeve 20 is rotatable relative to the outer tube 30 around the longitudinal axis L to a certain extent that permits to bring the tube assembly 10 into engagement with the polyaxial bone anchoring device 2. Moreover, in the assembled state of the tube assembly 10, the plunger 50 is configured to enter at least a portion of the polyaxial bone anchoring device 2 and to press onto the rod 3 from above if the rod 3 is present in the bone anchoring device 2. Furthermore, in the assembled state of the tube assembly 10, the outer tube 30 and the inner tube 40 are axially displaceable relative to each other along the longitudinal axis L to a certain extent that permits to move a portion of the polyaxial bone anchoring device 2 from an unlocking position of the bone anchoring element 4 to a locking position of the bone anchoring element 4 and vice-versa. The axial displacement of the inner tube 30 and the outer tube 40 relative to one another is effected by the actuator assembly 100 as explained in more detail below.

As shown in greater detail in Figs. 2, 4, 11 and 12, the outer tube 30 comprises a front end 30a and a rear end 30b opposite the front end 30a. The outer tube 30 comprises an internal channel extending between the front end 30a and the rear end 30b and configured to receive the inner tube 40 therein. A front portion of the outer tube 30 adjacent to the front end 30a may taper in a section 31 towards the front end 30a at an outer surface of the outer tube to reduce an overall space needed by the instrument when attached to the polyaxial bone anchoring device 2.

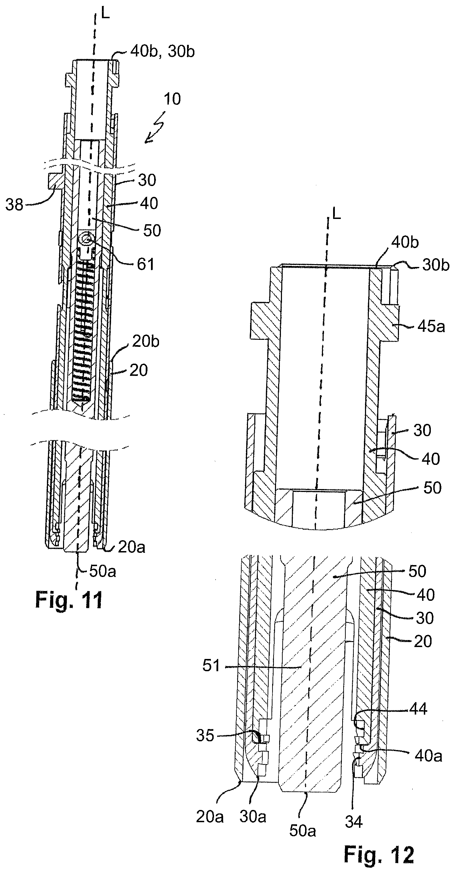

A recess 32 is formed that extends from the front end 30a of the outer tube 30 to a distance thereof. The recess 32 has a width in the circumferential direction that is larger than an upper width of the polyaxial bone anchoring device 2 and a height in the axial direction that permits the rod 3 as shown in Fig. 1 to extend therethrough and to be movable in an axial direction within the recess 32. As best seen in Fig. 12, at a distance from the front end 30a of the outer tube 30 an engagement structure 34 that is adapted to engage a corresponding engagement structure of the polyaxial bone anchoring device 2 is provided. The engagement structure 34 may be in the form of a circumferentially extending rib formed at the inner surface of the outer tube, which rib cooperates with a corresponding groove at the polyaxial bone anchoring device 2. At a distance from the engagement structure 34 towards the rear end 30b of the outer tube, a stepped portion or circumferentially extending shoulder 35 is provided at the inner surface of the outer tube that forms an abutment for the inner tube 40.

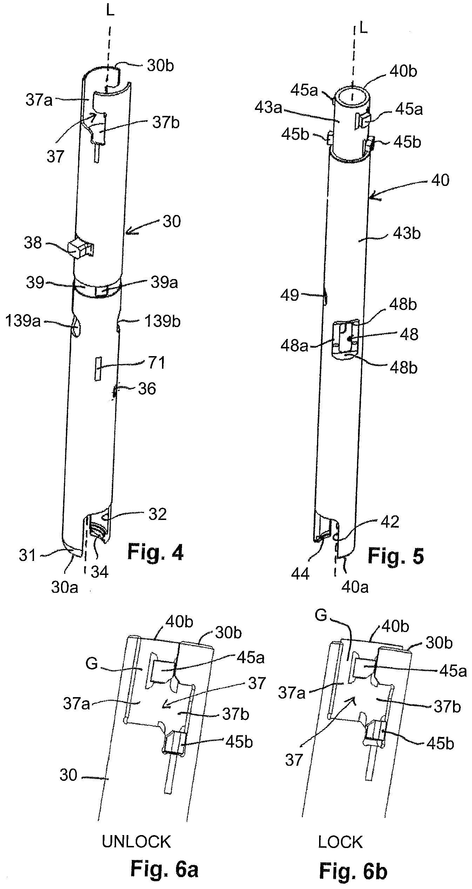

As best seen in Figs. 4 and 6a, 6b, adjacent to the rear end 30b of the outer tube 30 a cut-out or recess 37 is formed on each side of the longitudinal axis L. The recess 37 is open to the rear end 30b and extends to a distance from the rear end 30b. Each recess 37 has in a perspective view, seen in Figs. 6a and 6b, a substantially step-like shape forming a first recess area 37a that is open to the rear end 30b and adjacent thereto in a circumferential direction a second recess area 37b that may have a smaller width in the circumferential direction and extends from a distance from the rear end 30b farther down, i.e. towards the front end 30a compared to the first recess area 37a. The recess 37 has the function to accommodate driving portions of the actuator assembly and driven portions from the inner tube 40 therein. The orientation of the recess 37 is such that the recess 37 is substantially aligned in a circumferential direction with the engagement structure 34 at the front end 30a.

The corners formed between the first recess area 37a and the second recess area 37b may be rounded or slanted, as shown in Figs. 6a, 6b.

At a distance from the rear end 30b a protrusion 38 is formed at the outer surface of the outer tube 30, which protrusion 38 serves for engagement with an elongate slot of the actuator assembly 100. The protrusion 38 is located at a circumferential position substantially aligned with the recess 37 at the rear end 30b and the engagement structure 34 at the front end 30a.

At an axial distance from the protrusion 38 and further towards the front end 30a an attachment feature in the form of a circumferentially extending groove 39 and a recess 39a is provided in the outer tube 30. The circumferentially extending groove 39 is provided in the outer surface of the outer tube 30 and serves for receiving a clipping part 60, shown in Figs. 2 and 3, therein. The circumferentially extending groove 39 may extend along the entire circumference of the outer tube, or only a portion thereof that at least corresponds to the circumferential extension of the clipping part 60. **In** greater detail, the recess 39a is formed as a through-hole extending throughout the entire wall of the outer tube 30. The recess 39a serves for receiving a pin 61 provided at the inner surface of the clipping part 60, which pin 61 has a length that exceeds the thickness of the outer tube 30 and the inner tube 40 so that it can engage the plunger 50 in the assembled state of the tube assembly 10. A location of the recess 39a is at the circumferentially extending groove 39 at a circumferential position slightly offset from the middle of the recess 32 at the front end 30a.

At an axial distance from the groove 39 and further towards the front end 30a of the outer tube 30 two recess 139a, 139b are optionally provided in the wall of the outer tube 30. The recesses 139a, 139b are provided at opposite sides of the outer tube in a circumferential direction thereof, i.e., spaced apart at substantially 180° from one another, and each recess may be located at a circumferential position offset by about 90° from the recess 39a. The recesses 139a, 139b extend through the entire wall of the outer tube and may serve to attach additional navigation and/or sensor elements to the tube assembly, or to provide access to the inner tube 40. Each recess 139a, 139b may have a substantially elongate or oval contour when viewed in the plane of the outer surface of the outer tube, with a longer axis of the contour extending in the circumferential direction of the outer tube 30.

At an axial distance from the groove 39 and/or the recesses 139a, 139b and further towards the front end 30a of the outer tube 30 a marking 71 may be provided that serves to indicate a rotational position of the outer sleeve 20 with respect to the outer tube 30. The marking 71 may be an elongate stripe or a straight line extending in the axial direction of the outer tube 30. The marking 71 may be located at a circumferential position substantially aligned with the recess 39a of the attachment feature.

At an axial distance from the marking 71 and further towards the front end 30a of the outer tube 30 two engagement features in the form of recesses 36 are provided in the outer surface of the outer tube 30, only one of which recesses 36 can be seen in the figures. The recesses 36 are spaced apart in a circumferential direction of the outer tube and serve for receiving corresponding engagement features, such as engagement latches, of the outer sleeve 20 in the respective rotational positions of the outer sleeve 20, as explained in more detail below.

Turning now again to Fig. 2 and additionally to Figs. 5, 11 and 12, the inner tube 40 comprises a front end 40a and a rear end 40b and an internal channel extending from the front end 40a at least along a section of the inner tube towards the rear end 40b and configured to receive the plunger 50 therein. An inner diameter of the inner tube 40, i.e. a diameter of its internal channel, is such that a fixation element such as a set screw or a further instrument can pass through.

In a front portion adjacent to the front end 40a of the inner tube 40 a recess 42 is formed that substantially corresponds to the recess 32 of the outer tube 30, such that when the inner tube 40 is in the outer tube 30 the recesses 32 and 42 are aligned (see e.g. Fig. 3). At a distance from the front end 40a an engagement structure 44 is provided that is configured to cooperate with a corresponding engagement structure at the polyaxial bone anchoring device 2. The engagement structure 44 in the embodiment shown is a circumferentially extending groove provided in the inner surface of the inner tube 40, which groove may cooperate with a circumferentially extending rib at the polyaxial bone anchoring device 2.

In an upper region 43a adjacent the rear end 40b of the inner tube 40 first protrusions 45a, 45b are formed at the outer surface thereof, which first protrusions serve as driven portions that are driven by driving portions of the actuator assembly 100 to displace the inner tube 40 relative to the outer tube 30. Two sets of first protrusions 45a, 45b are provided at opposite sides from the longitudinal axis L in the circumferential direction of the inner tube 40. As depicted in Figs. 2, 5 and 6a, 6b, one protrusion 45a is located at a first distance from the rear end 40b and the other protrusion 45b is provided at a second distance greater than the first distance from the rear end 40b and is circumferentially offset from and adjacent to the protrusion 45a. The protrusions 45a, 45b may have approximately a square-shaped contour, optionally with slanted edges, and may have such a height in the radial direction that they are substantially flush with the outer cylindrical surface of the outer tube 30 when the inner tube 40 and the outer tube 30 are assembled. To achieve this, the inner tube 40 may have a reduced outer diameter in the upper region 43a that comprises the first protrusions 45a, 45b as compared to an outer diameter of the inner tube in a lower region 43b of the inner tube. As depicted in greater detail in Figs. 6a and 6b, in the assembled state, the protrusion 45a that is closer to the rear end 40a of the inner tube 40 is located in the first recess area 37a of the recess 37 of the outer tube 30 and the second protrusion 45b is located in the lowermost portion of the second recess area 37b of the recess 37 of the outer tube 30. The distance of the protrusions 45a, 45b in the circumferential direction is such that there is in the assembled state a gap G between the first protrusion 45a and an opposing sidewall of the recess 37 that serves for entering driving portions of the actuator assembly into the recess 37. The inner tube 40 may be assembled with the outer tube 30 by first introducing the lower protrusion 45b into the recess 37, rotating the inner tube 40 relative to the outer tube 30 and then entering the upper protrusion 45a into the recess 37.

At an axial distance from the upper region 43a of the inner tube 40 and further towards the front end 40a a recess 49 is provided. The recess 49 is formed as a through-hole extending through the entire wall of the inner tube 40 and serves for receiving the pin 61 of the clipping part 60. Moreover, the recess 49 is located at an axial and circumferential position of the inner tube that corresponds to the axial and circumferential position of the recess 39a of the outer tube 30 in the assembled state of the tube assembly. In greater detail, the recess 49 has an elongate, e.g. oval, shape with a long axis of the elongate shape extending in the axial direction of the inner tube 40 to enable axial displacement of the inner tube 40 and the outer tube 30 relative to one another in the direction of the longitudinal axis L even when the pin 61 of the clipping part 60 extends from the clipping part 60 through both recesses 39a, 49 of the outer and inner tubes.

At an axial distance from the recess 49 of the inner tube optionally a recess 48 is provided that extends through the entire wall of the inner tube 40. A region of the wall of the inner tube around the recess 48 may be locally thinned so that a contour 48a of the recess 48 at the outer surface of the inner tube is larger than the contour of the recess 48 at the inner surface thereof, and/or to provide inclined wall portions 48b at the upper and/or lower end of the recess 48 in the axial direction. The recess 48 may have a substantially elongate or oval contour, with a long axis of the contour extending in the axial direction of the inner tube 40, i.e., in the direction of the longitudinal axis L. The recess 48 is located at an axial and circumferential position of the inner tube 40 that corresponds to the axial and circumferential position of the recess 139a or 139b of the outer tube 30 in the assembled state of the tube assembly. The inner tube 40 may be provided with two such recesses 48 located at opposite sides of the inner tube in the circumferential direction thereof.

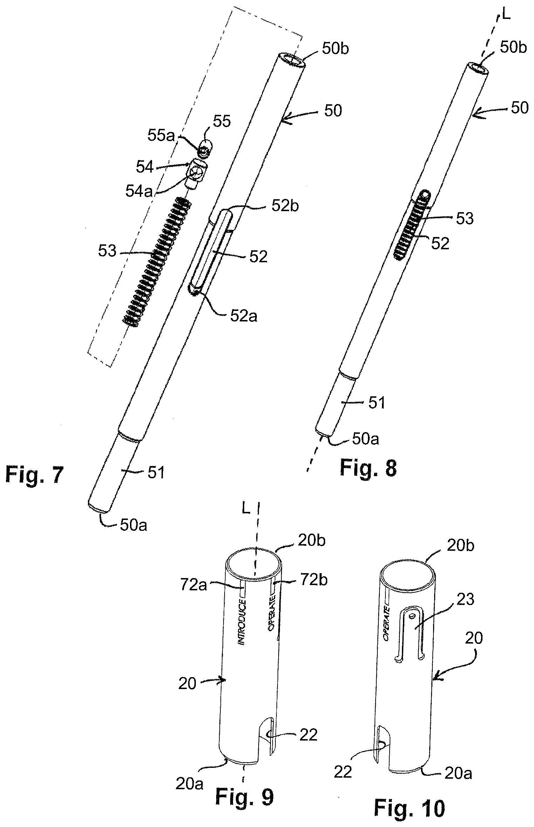

Turning again to Fig. 2 and additionally to Figs. 7, 8, 11 and 12, the plunger 50 comprises a front end 50a and a rear end 50b. An outer diameter of the plunger 50 is such that the plunger 50 can be accommodated within the internal channel of the inner tube 40 of the tube assembly. A front region 51 of the plunger 50 adjacent to the front end 50a may be provided with a reduced outer diameter so that a circumferentially extending gap is provided between the outer surface of the front region 51 of the plunger 50 and the inner surface of the inner tube 40 in the assembled state of the tube assembly at least in a portion along the longitudinal axis L as shown in Figs. 11 and 12. The gap may be sized to accommodate a portion of the receiving part of the polyaxial bone anchoring device 2 therein and to enable the front region 51 of the plunger 50 to enter the receiving part. In a rear region of the plunger 50, the plunger 50 may be provided with an outer cylindrical surface that contacts the inner surface of the inner tube when the inner tube and the plunger are assembled.

Located axially between the rear end 50b of the plunger 50 and its front region 51, an elongate opening 52 is provided that penetrates the plunger 50 in a direction perpendicular to the longitudinal axis L. The opening 52 extends between a first end 52a closer to the front end 50a of the plunger and a second end 52b closer to the rear end 50b of the plunger. The opening 52 is sized to accommodate an elastic element, such as a coil spring 53, therein. Between the second end 52b of the opening 52 and the coil spring 53 is located an attachment feature in the form of a cylindrical attachment element 54 provided with a through-hole 54a. The through-hole 54a of the attachment element 54 accommodates a sleeve 55 provided with a through-hole 55a configured to receive the pin 61 of the clipping part 60 (see Figs. 2, 3) therein to attach the plunger 50 to the inner and outer tubes 40, 30.

In the assembled state of the tube assembly, as shown in Figs. 3, 11 and 12, the plunger 50 extends with its front end 50a axially into the recesses 32, 42 of the outer and inner tubes 30, 40, and preferably axially protrudes with its front end 50a out of the outer tube 30 and/or the outer sleeve 20. When attaching the tube assembly 10 to the polyaxial bone anchoring device 2 while the rod 3 is present in the receiving part, as shown in Fig. 1, the plunger 50 is axially displaced towards the rear end 40b of the inner tube 40 due to the presence of the rod 3, which causes the coil spring 53 to compress within the elongate opening 52 and to thus generate a counter-force acting upon the plunger 50 in the downward direction, i.e., towards the front end 40a of the inner tube, thus pressing the rod 3 downward in the receiving part.

Turning again to Figs. 2 and 3 and with further reference to Figs. 9 to 12, the outer sleeve 20 comprises a front end 20a rear end 20b and an internal channel extending from the front end 20a to the rear end 20b. An inner diameter of the outer sleeve 20, i.e., of the internal channel, is such that a front portion of the outer tube 30 can be received therein.

In a front region adjacent to the front end 20a a recess 22 is formed that extends from the front end 20a of the outer sleeve 20 to a distance thereof. The recess 22 has a height in the axial direction that permits the rod 3 to extend therethrough and to be movable in an axial direction within the recess 22 (Fig. 1). A width of the recess 22 in the circumferential direction substantially corresponds to or is slightly larger than a diameter of the rod 3 and is smaller than the widths of the recesses 32 and 42 of the outer tube 30 and inner tube 40, respectively.

An axial length of the outer sleeve 20 between the front end 20a and the rear end 20b may be shorter than an axial length of the inner tube 40 and the outer tube 30. The axial length of the outer sleeve 20 may be such that the rear end 20b is substantially located in a region of the marking 71 at the outer tube 30 when the outer sleeve 20 is assembled with the outer tube 30.

At a distance from the rear end 20b of the outer sleeve 20 an engagement feature in the form of a latching tab 23 is provided that is configured to selectively engage one of the two recesses 36 provided at the outer surface of the outer tube 30. The engagement of the latching tab 23 with the respective recess 36 of the outer tube 30 attaches the outer sleeve 20 to the outer tube 30 at a specific rotational position. Specifically, engagement of the latching tab 23 with a first one of the recesses 36 defines a first rotational position of the outer sleeve with respect to the outer tube, and engagement of the latching tab 23 with a second one of the recesses 36 defines a second rotational position of the outer sleeve with respect to the outer tube.

Markings 72a, 72b are provided at the outer surface of the outer sleeve 20 adjacent the rear end 20b that indicate a position and/or a function of the tube assembly 10. The markings 72a, 72b are offset from one another in the circumferential direction of the outer sleeve 20 so that when the latching tab 23 engages the first recesses 36 of the outer tube 30 in the first rotational position, a first marking 72a of the outer sleeve 20 is circumferentially aligned with the marking 71 of the outer tube 30, and when the latching tab 23 engages the second recesses 36 of the outer tube 30 in the second rotational position, a second marking 72b of the outer sleeve 20 is circumferentially aligned with the marking 71 of the outer tube 30. The first marking 72a may be provided with a function indication "INTRODUCE", and the second marking 72b may be provided with a function indication "OPERATE".

As explained in more detail below, when the outer sleeve 20 is in the first rotational position indicated by the "INTRODUCE" indication where the first marking 72a of the outer sleeve 20 is aligned with the marking 71 of the outer tube 30, the tube assembly can be placed onto the receiving part of the polyaxial bone anchoring device 2 and is removable therefrom. In the second rotational position of the outer sleeve 20, indicated by the "OPERATE" indication where the second marking 72b of the outer sleeve 20 is aligned with the marking 71 of the outer tube 30, the tube assembly engages the receiving part of the polyaxial bone anchoring device 2 and is not removable therefrom.

The widths of the recesses 32 and 42 in the circumferential direction of the outer tube 30 and inner tube 40, respectively, exceed the width of the recess 22 of the outer sleeve 20 so that in both the "INTRODUCE" position and the "OPERATE" position of the outer sleeve 20 with respect to the tubes 30, 40 all recesses 22, 32, 42 of the outer sleeve 20 and the tubes 30, 40 overlap in the circumferential direction so as to allow a rod to pass therethrough.

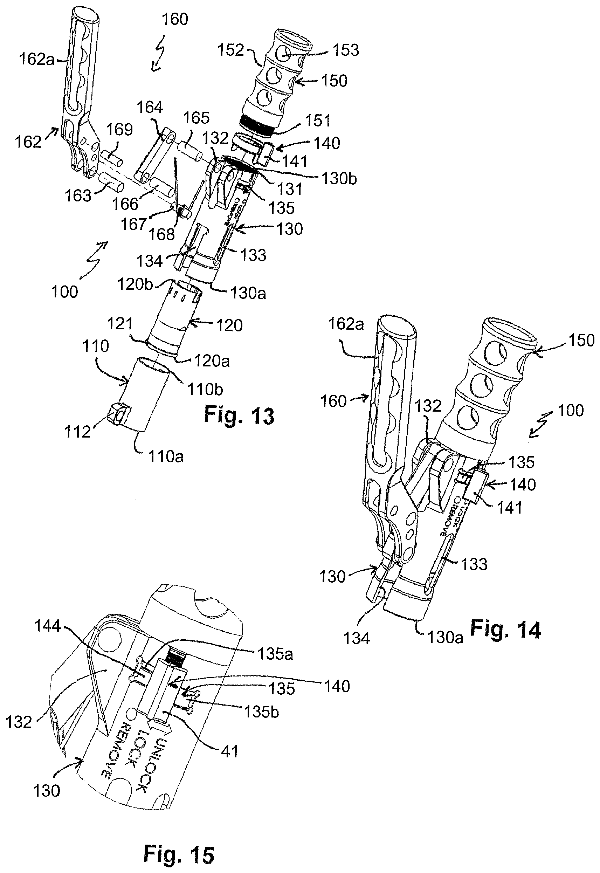

Next, the actuator assembly will be described referring in greater detail to Figs. 13 to 19. The actuator assembly 100 comprises a first inner sleeve 110, a second inner sleeve 120, an outer sleeve 130, an adjustment member 140, a handle portion 150 and a lever assembly 160 in the form of a toggle lever.

The outer sleeve 130 has a front end 130a and a rear end 130b. An internal thread 131 is provided at or near the rear end 130b that allows to connect the handle portion 150 to the outer sleeve 130.

Adjacent to the rear end 130b a first hinge 132 is provided that serves for attachment of one of the lever arms of the lever assembly 160, more specifically that provides one rotation point of the toggle lever. A plurality of elongate openings 133 may be provided circumferentially that facilitate cleaning. In addition, an elongate slot 134 extends from the front end 130a to a distance from the front end in an axial direction at the same circumferential position as the first hinge 132. The slot 134 permits a second hinge provided at the first inner sleeve 110 and the protrusion 38 provided at the outer tube 30 of the tube assembly 10 to protrude therethrough.

At around 90° in a circumferential direction from the first hinge 132 an opening or recess 135 is provided in the wall of the outer sleeve 130 that permits an adjustment lever 141 of the adjustment member 140 to protrude there through. As shown in more detail in Fig. 15, the opening 135 is closed to the rear end 130b of the outer sleeve 130. The opening 135 has in a perspective view a substantially step-like shape forming a first opening area 135a and adjacent thereto in a circumferential direction a second opening area 135b, wherein the first and second opening area 135a, 135b have the same extension in the axial direction and the first opening area 135a is provided at a smaller distance from the rear end 130b than the second opening area 135b.

A width of the second opening area 135b in the circumferential direction is sized so as to permit the adjustment lever 141 of the adjustment member 140 described below to move along the circumferential direction of the outer sleeve to a certain extent. The distance between the rear end 130b of the outer sleeve 130 and the first opening area 135a is such that the adjustment lever 141 of the adjustment member 140 can be received in the first opening area only when the handle portion 150 is moved farther away from the rear end 130b, e.g., screwed out of the outer sleeve 130, to provide for additional space to move the adjustment lever 141 upwards from the second opening area 135b into the first opening area 135a.

At an axial distance from the opening 135 and further towards the front end 130a of the outer sleeve function indications "REMOVE", "LOCK" and "UNLOCK" may be provided, for example additionally with a marking provided at the "REMOVE" indication to indicate a remove position of the actuator assembly 100, and/or an arrow that indicates the direction of rotation of the adjustment member 140 to achieve a locking position and an unlocking position. The "REMOVE" indication is provided axially below the first opening area 135a and the "LOCK" and "UNLOCK" indications are provided axially below the second opening area 135b of the outer sleeve 130 at different circumferential positions thereof.

The handle portion 150 comprises a threaded projection 151 that cooperates with the internal thread 131 of the outer sleeve 130 to allow screwing the handle portion 150 into the outer sleeve 130. The handle portion 150 may further comprise a gripping structure 152 such as circumferential lobes or crests that facilitates gripping. A plurality of through-holes 153 may also be provided at the handle portion 150 to facilitate cleaning.

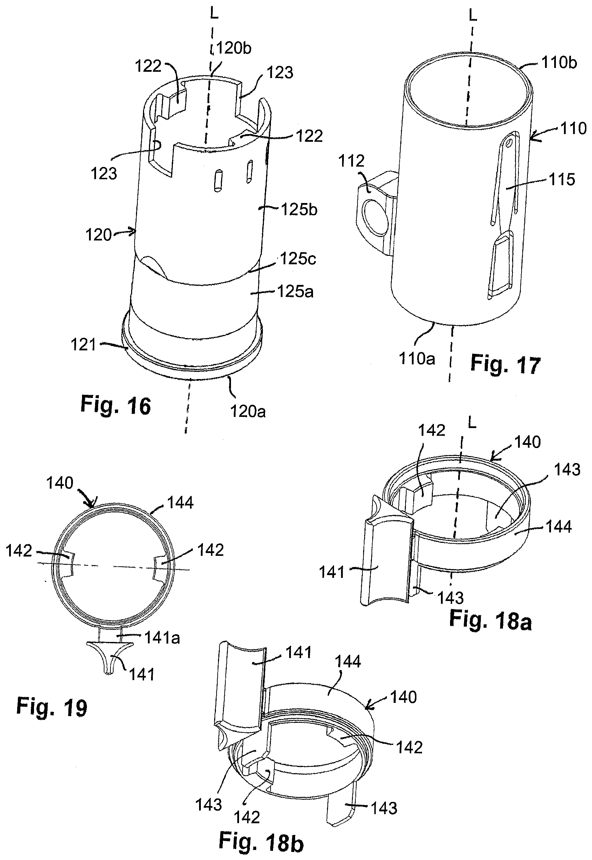

Referring additionally to Fig. 17, the first inner sleeve 110 comprises a front end 110a and a rear end 110b and has an axial length that is shorter than the axial length of the outer sleeve 130 and an outer diameter that is smaller than an inner diameter of the outer sleeve 130 so that the first inner sleeve 110 can be fully accommodated within the outer sleeve 130. Adjacent to the front end 110a and at the same circumferential position as the first hinge 132 of the outer sleeve 130, a second hinge 112 is provided at the outer surface of the first inner sleeve 110. In the assembled state of the actuator assembly 100, the second hinge 112 protrudes outward from the first inner sleeve 110 through the slot 134 of the outer sleeve 130. The second hinge 112 serves as a second rotation point of the toggle lever.

Moreover, the first inner sleeve 110 comprises a latching feature in the form of a latching tab 115 that locally protrudes into the interior channel of the first inner sleeve 110 and serves for engagement with the second inner sleeve 120.

The second inner sleeve 120 that is shown in more detail in Fig. 16 comprises a front end 120a and a rear end 120b and has an outer diameter that is smaller than an inner diameter of the first inner sleeve 110 so that the second inner sleeve 120 can be accommodated in the first inner sleeve 110. Adjacent to the front end 120a the second inner sleeve 120 comprises a circumferentially extending protrusion 121 at its outer surface that forms an abutment for the front end 110a of first inner sleeve 110. In a front region 125a adjacent the circumferentially extending protrusion 121 the outer diameter of the second inner sleeve is slightly reduced as compared to a rear region 125b adjacent the rear end 120b, thus forming an abutment or shoulder 125c at its outer surface configured to be engaged by the latching tab 115 of the first inner sleeve 110. By the engagement of the shoulder 125c by the latching tab 115 and the front end 110a of first inner sleeve 110 abutting at the circumferentially extending protrusion 121 of the second inner sleeve 120, the first and second inner sleeves are fixed relative to each other with respect to their axial position.

Adjacent to the rear end 120b two inner protrusions 122 are formed on the inner surface of the second inner sleeve 120, the protrusions 122 being provided at opposite sides of the longitudinal axis, i.e. offset by 180° in the circumferential direction. The protrusions 122 form second protrusions compared to the first protrusions of the tube assembly 10. They may have a substantially square-shaped contour and serve as driving portions for transferring the actuating movement of the lever assembly 160 to the tube assembly 10 as explained in detail below. Moreover, two recesses 123 are provided at the rear end 120b, wherein the recesses are offset by 180° from one another in the circumferential direction and are arranged at substantially 90° with respect to the protrusions 122. Each recess 123 extends from the rear end 120b to a distance thereof and may have a substantially square-shaped contour. The recesses 123 serve to receive extending portions of the adjustment member 140 therein.

The adjustment member 140 is shown in more detail in Figs. 18a, 18b and 19. The adjustment member 140 comprises an annular ring 144 sized so as to be received within the outer sleeve 130. At the outer surface of the annular ring 144 the adjustment lever 141 is provided that is connected to the annular ring 144 via a protrusion 141a. The protrusion 141a has a size that permits it to protrude through the opening 135 to the outside of the outer sleeve 130 when the adjustment member 140 is received within the outer sleeve 130. The adjustment lever 141 is thus accessible from the outside of the outer sleeve for manual actuation by a user.

The adjustment member 140 comprises two inner protrusions 142 at the inner surface of the annular ring 144 that are offset from one another by 180°. The inner protrusions 142 are similar to the inner protrusions 122 of the second inner sleeve 120, i.e. have a substantially square-shaped contour. The inner protrusions 142 protrude from the inner surface of the annular ring 144 to locally narrow its inner width.

Furthermore, two extending portions 143 are provided at the lower side of the of the annular ring 144 that protrude away from the annular ring 144 in the downward direction, i.e., towards the front end 130a of the outer sleeve 130 in the assembled state of the actuator assembly 100. The extending portions 143 are offset from one another by 180° in the circumferential direction of the annular ring 144 and are arranged at substantially 90° with respect to the protrusions 142. The extending portions 143 have a shape similar to the shape of the recesses 123 of the second inner sleeve 120, i.e., each extending portion 143 may have a substantially square-shaped contour, so that each of the extending portions 143 can be accommodated at least partially within the respective recesses 123 of the second inner sleeve 120. A width of the extending portion 143 in the circumferential direction corresponds to the width of the recess in the circumferential direction. Thus, when the adjustment lever 141 is rotated in the circumferential direction within the opening 135 of the outer sleeve 130, the adjustment member 140 rotates in the circumferential direction together with the second inner sleeve 120 that is engaged by the adjustment member via the extending portions 143.

In the assembled state of the actuator assembly 100, the extending portions 143 of the adjustment member 140 engage the recesses 123 of the second inner sleeve 120 so that the inner protrusions 142 of the adjustment member 140 are circumferentially aligned with the inner protrusions 122 of the second inner sleeve 120. Like the inner protrusions 122 of the second inner sleeve 120, the inner protrusions 142 of the adjustment member 140 are second protrusions when compared to the first protrusions 45a, 45b of the tube assembly 10 and also serve as driving portions that are configured to transfer the actuating movement of the actuator assembly 100 to the tube assembly 10.

Turning again to Figs. 13 and 14, the lever assembly 160 comprises a first lever arm 162 that is hingedly connected at one end via a pin 163 to the second hinge 112 provided at the first inner sleeve 110. A longer end portion or grip portion 162a of the first lever arm 162 may have a gripping structure at its side facing away from the sleeves 110, 120, 130 to facilitate actuation with the hand. A second lever arm 164 of the lever assembly 160 is on one side hingedly connected via a pin 165 to the first hinge 132 and at its second end via a pin 166 hingedly connected to the first lever arm 162. The latter connection provides the third rotation point of the toggle lever. In the embodiment shown, the second lever arm 164 is shorter than the first lever arm 162. The first lever arm 162 has a slightly angled shape. More specifically, a length of the grip portion 162a of the first lever arm 162 is such that the height position of the first lever arm substantially corresponds to the height position of the handle portion 150.

Moreover, an elastic member in the form of a spring 168 is provided at the first lever arm 162. The spring 168 is supported at the first lever arm 162 by a pin 167 and abuts with a first end portion thereof against another pin 169 provided at the first lever arm 162 and abuts with a second end portion against the outer sleeve 130. Thus, if the first lever arm 162 is moved towards the handle portion 150 into a more upright position, i.e., into a straight configuration of the lever assembly 160 where the first lever arm 162 and the second lever arm 164 are more parallel or less angled (not shown in the figures), the spring 168 generates a force that pushes the first lever arm 162 away from the handle portion 150 and back into its initial position that is shown in Fig. 14, which is an angled configuration of the lever assembly 160 where the first lever arm 162 and the second lever arm 164 are angled with respect to each other and the grip portion 162a extends in an angled manner away from the handle portion 150. This angled configuration is a default configuration of the lever assembly 160 due to the preload generated by the spring 168.

When the first lever arm 162 is pushed towards the handle portion 150, the lever assembly 160 assumes the straight configuration, where the first lever arm 162 and the second lever arm 164 are more parallel or less angled (not shown in the figures). This causes the first inner sleeve 110 to abut against the circumferentially extending protrusion 121 of the second inner sleeve 120 and both inner sleeves 110, 120 are pushed downward towards the front end 130a of the outer sleeve 130, whereby the inner protrusions 122 of the second inner sleeve 120 axially move away from the inner protrusions 142 of the adjustment member 140 so that a distance between the protrusions 122 of the second inner sleeve 120 and the protrusions 142 of the adjustment member 140 increases in the axial direction. The axial movement of the two protrusions 122 and 142 relative to one another, which are the driving portions of the actuator assembly 100, is transferred onto the protrusions 45a, 45b of the tube assembly 10, i.e., the driven portions, to displace the inner tube 40 of the tube assembly 10 relative to the outer tube 30 as described in greater detail below.

The instrument is made of a body-compatible material. In particular, titanium or stainless steel may be such a suitable material, but other materials could also be used as long as they are body compatible.

Referring now again to Figs. 1 to 3, 6a, 6b and 13 to 15, mounting of the tube assembly 10 and the actuator assembly 100 and the actuation of the tube assembly 10 by the actuator assembly 100 will be explained.

Mounting of the actuator assembly 100 is accomplished as follows. The plunger 50 is within the inner tube 40, and the inner tube 40 is within the outer tube 30, with the clipping part 60 received within the circumferentially extending groove 39 so that its pin 61 extends through the inner and outer tubes into the through-hole 55a of the plunger 50 to hold the plunger 50 within the tubes. The first protrusions 45a, 45b of the inner tube 40 are in the respective recess areas 37a, 37b of the recess 37 of the outer tube 30.

The outer sleeve 20 is then mounted onto the outer tube 30 by sliding the outer sleeve 20 onto the outer tube 30. Therein, the outer sleeve 20 is in the first rotational position relative to the outer tube 30, in which position the "INTRODUCE" indication of the first marking 72a of the outer sleeve 20 is circumferentially aligned with the marking 71 of the outer tube 30. The outer sleeve 20 is slid onto the outer tube 30 until the latching tab 23 of the outer sleeve 20 engages the recesses 36 of the outer tube 30.

The adjustment lever 141 of the actuator assembly 100 is in a circumferential position that indicates the "REMOVE" position, i.e., it is within the first opening area 135a of the opening of the outer sleeve 130, which is enabled by unscrewing the handle portion 150 from the outer sleeve 130 to a certain extent. The actuator assembly 100 is mounted onto the tube assembly 10 in such an orientation that the protrusion 38 at the outer surface of the outer tube 30 enters the elongate slot 134 of the outer sleeve 130 of the actuator assembly. In this orientation of the actuator assembly 100, the second protrusions 122, 142 of the actuator assembly, which are circumferentially aligned with one another, are allowed to pass downward through the gap G of the tube assembly 10, which gap G is formed between the first protrusion 45a of the inner tube 40 and an opposing sidewall of the recess 37 of the outer tube 30. Removing of the actuator assembly 100 from the tube assembly 10 is accomplished in the same position, wherein the second protrusions 122, 142 of the actuator assembly move upwards through the gap G of the tube assembly.

The locking configuration of the instrument 1 is attained as follows. First, the adjustment lever 141 of the actuator assembly 100 rotated counterclockwise and moved downwards into the second opening area 135b of the opening of the outer sleeve 130 and the handle portion 150 is fully screwed into the outer sleeve 130 to prevent the adjustment lever 140 to unintentionally attain the "REMOVE" position where it is received in the first opening area 135a. When received in the second opening area 135b, the adjustment lever 141 can be rotated in the circumferential direction to selectively align with the "LOCK" indication or with the "UNLOCK" indication. When the adjustment lever 141 is moved into the "LOCK" position, the second protrusions 122, 142 of the actuator assembly 100 move into the first recess area 37a of the tube assembly 10 below the upper first protrusions 45a of the inner tube 40. When the lever assembly 160 is actuated by pressing the first lever arm 162 in the direction of the handle portion 150 so that the straight configuration (not shown in the figures) is attained, the second protrusions 122, 142 become spaced apart from each other as explained above, thereby generating or increasing a gap between them in the axial direction. As the lower wall of the recess 37 provides an abutment for the second protrusion 122 of the inner sleeve 120, the second protrusion 142 of the adjustment member 140 presses the first protrusion 45a of the inner tube 40 upward towards the rear end 30b of the outer tube 30 and the first protrusion 45b also moves upward with respect to the outer tube (Fig. 6b). As a consequence, the front ends 30a, 40a of the tubes are axially displaced with respect to each other such that the distance between the engagement structures of the inner and outer tube increases.

The unlocking configuration is attained by moving the adjustment lever 141 of the actuator assembly 100 into the "UNLOCK" position. The second protrusions 122, 142 of the actuator assembly 100 enter the second recess area 37b of the tube assembly 10 above the lower first protrusion 45b. When the lever assembly 160 is actuated by pressing the first lever arm 162 in the direction of the handle portion 150 so that the straight configuration (not shown in the figures) is attained, the second protrusions 122, 142 become spaced apart from each other so that the second protrusion 122 of the inner sleeve 120 pushes down the first protrusion 45b of the inner tube 40. The opposite (upper) wall of the recess 37 serves as an abutment for the second protrusion 142 of the adjustment member 140 (Fig. 6a). As a consequence, the front ends 30a, 40a of the tubes are axially displaced with respect to each other such that the distance between the engagement structures of the inner and outer tube becomes smaller.

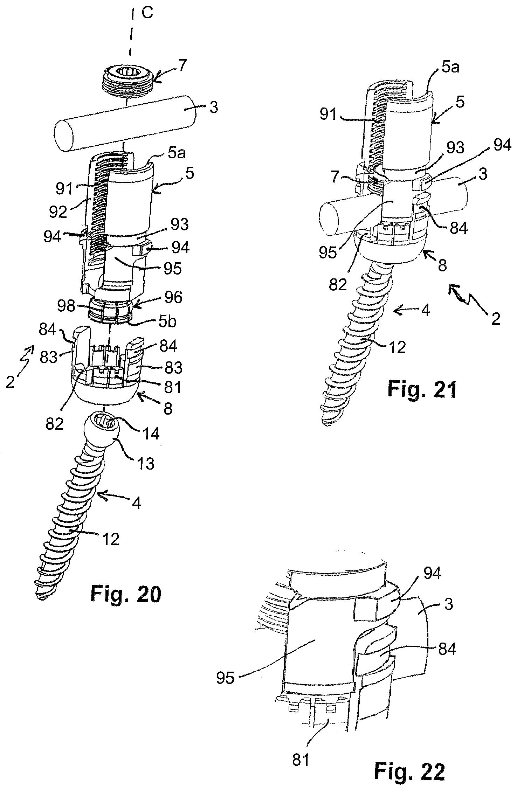

Next, with reference to Figs. 20 to 22, a polyaxial bone anchoring device 2 that is suitable for use with the instrument 1 will be explained. The polyaxial bone anchoring device 2 according to an embodiment comprises a bone anchoring element 4 having a shank 12 and a head 13 with a spherically-shaped outer surface portion. The bone anchoring element 4 may be a bone screw with a threaded shank. The head 13 may have a recess 14 is provided for engagement with a tool, such as a driver. A receiving part 5 is provided for receiving the head 13 and connecting the bone anchoring element 4 via the head 13 to a rod 3. In addition, a fixation element 7 in the form of an inner screw or a set screw may be provided for fixing the rod 3 in the receiving part 5. Also, the bone anchoring device 2 includes a locking ring 8 for locking the head 13 in the receiving part 5.

The receiving part 5 has a first or upper end 5a and a second or lower end 5b. Adjacent to the upper end 5a, a rod receiving portion 90 is provided and adjacent to the lower end 5b a head receiving portion 96 is provided. The rod receiving portion 90 is substantially cylindrical and comprises a coaxial bore 91 that extends from the upper end 5a into the head receiving portion 96. The bore 91 comprises an internal thread in at least a region thereof for receiving the fixation element 7. A substantially U-shaped recess 92 that forms a channel for receiving the rod 3 extends from the upper end 5a to almost the beginning of the head receiving portion 96. At a distance from the upper end 5a a groove or otherwise weakened section 93 may be provided that allows to break-off the upper portions of the receiving part 5 formed by the U-shaped recess that serve as extended tabs. By means of the long extended tabs it is possible to manipulate the polyaxial bone anchoring device 2 with an inserted rod 3 that is at a higher position compared to the final position so that, for example, a vertebra can be pulled against the rod.

At an outer surface of the rod receiving portion 90 an engagement structure for engagement with the tube assembly 10 is provided. The engagement structure may comprise circumferentially extending ribs 94. The ribs 94 are arranged asymmetrical with respect to a plane including a central axis C of the receiving part 5 and a channel axis of the substantially U-shaped recess 92. That means, a first rib 94 starts at the U-shaped recess 92 on one side and extends to a distance around the receiving part and the second rib 94 starts at the opposite side of the U-shaped recess relative to the central axis C and extends from there to a distance around the receiving part 5. Thereby, a rib-free surface 95 is formed on each side from the U-shaped recess.

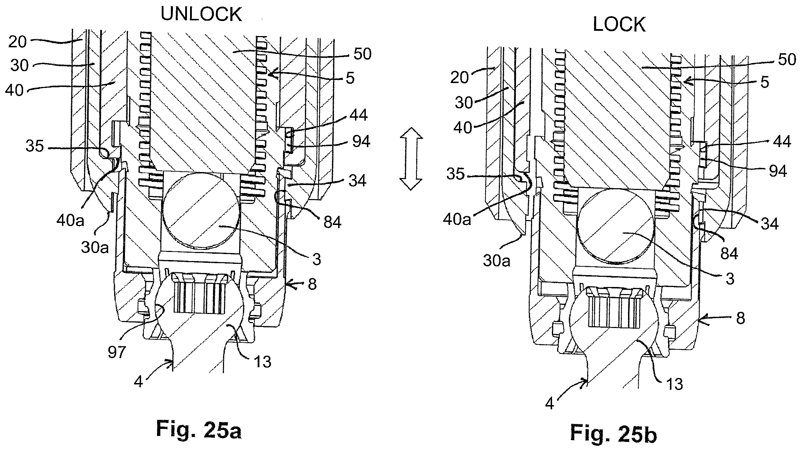

The head receiving portion 96 has a substantially cap-like shape with a hollow substantially spherical interior portion 97 (see Figs. 25a, 25b) for pivotably receiving the head 13 therein. A plurality of slits 98 render the head receiving portion flexible so that when pressure is exerted onto the head receiving portion by the locking ring 8, the head 13 can be clamped and finally locked.

The locking ring 8 is designed to encompass the head receiving portion 96 and has an internal surface structure that allows to achieve in corporation with the head receiving portion a full locking of the head 13 in the head receiving portion 96 when the locking ring 8 in its lowermost position and a pre-locking when the locking ring 8 is in a position slightly above the lowermost position which allows still pivoting of the head 13 in the head receiving portion but prevents removal of the head 13 from the head receiving portion 96.

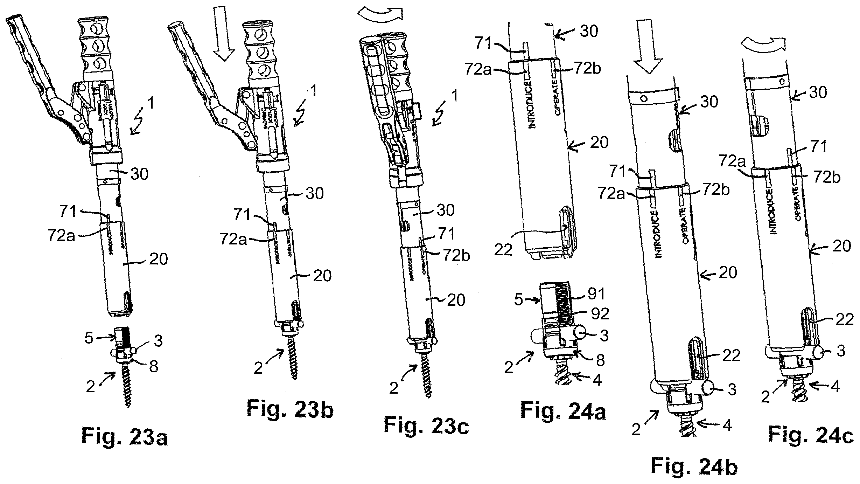

The locking ring 8 further has plurality of upstanding flexible sections 81 that may serve for engagement with the receiving part to preliminarily hold the locking ring 8 in a pre-locking position. Also, two opposite projections 82 are provided at an upper side of the locking ring that serve for supporting the rod 3. In the embodiment shown, the locking ring 8 also comprises two upstanding arms 83 that are asymmetrically with respect to a plane that extends through the central axis C and through the middle of the projections 82 in the same manner as the ribs 94 of rod receiving portion 90 are arranged. At an upper end of the arms 83 an engagement structure in the form of grooves 84 is provided that is configured to be engaged by the tube assembly 10 of the instrument 1. As depicted in Figs. 21 and 24, in the assembled state of the polyaxial bone anchoring device 2, the engagement structure in the form of the ribs 94 on the receiving part 5 and the engagement structure in the form of the grooves 83 on the locking ring 8 are circumferentially aligned, leaving the rib-free surface 95 of the head receiving portion 90 exposed. The upstanding arms 83 of the locking ring 8 with the engagement structure 84 at or near their upper end facilitate finding of the engagement structure with the instrument.

In the following, use of the instrument will be explained (not claimed). With further reference to Figs. 23a to 24c, first the steps of attaching the instrument 1 to the polyaxial bone anchoring device 2 are explained. The bone anchoring element 4 may be implanted in a vertebra or other bone. The locking ring 8 is in an axial position with respect to the receiving part 5 in which it does not fully lock the head but the head 13 is prevented from removal from the head receiving portion 96. The rod 3 is inserted into the substantially U-shaped recess 92. Due to the extended tabs, the rod 3 is still movable not only along the rod axis but also in an axial direction.

The tube assembly 10 of the instrument 1 is in the first rotational position of the outer sleeve 20 with respect to the outer tube 30, which position is indicated by the "INTRODUCE" indication where the first marking 72a of the outer sleeve 20 is aligned with the marking 71 of the outer tube 30, as depicted in Figs. 23a and 24a. Moreover, the engagement structure 34 of the outer tube 30 and the engagement structure 44 of the inner tube 40 have their closest distance from one another which is defined by an abutment of the front portion 40a of the inner tube 40 against the inner shoulder 35 of the outer tube 30 (see Fig. 12). In this configuration, the instrument 1 is placed onto the receiving part 5 of the bone anchoring device 2 and moved downward in such an orientation that the recess 22 of the outer sleeve 20 is aligned with the substantially U-shaped recess 92 of the receiving part 5 that forms a channel for the rod 3. This permits the instrument 1 to be placed onto the receiving part 5 with the rod 3 received therein. In this orientation of the instrument 1, the engagement structures 34 and 44 of the outer tube 30 and the inner tube 40 are circumferentially aligned with the rib-free outer surface portion 95 of the receiving part 5. The outer sleeve 20 with its recess 22 assists in placement of the instrument 1 onto the receiving part in the correct orientation. Moreover, the plunger 50 of the tube assembly enters the coaxial bore 91 of the receiving part, which also assists in placing the instrument onto the receiving part and pushes an inserted rod 3 downward within the U-shaped recess 92. Hence, both the outer sleeve 20 and the plunger 50 may serve as alignment members that assist in attaching the tube assembly 10 to the bone anchoring device 2. This facilitates handling of the instrument, because the instrument can be placed onto the receiving part with the recess 22 of the outer sleeve 20 aligned with the rod that is already inserted into the receiving part. The recess 22 thus provides an indication of the correct mounting position to the user.

Next, as shown in Figs. 23b, 24b, the engagement structures 34 and 44 of the tube assembly 20 of the instrument move along the rib-free surface portions 95 of the receiving part 5 to a position where the engagement structures in form of the ribs 34 of the outer tube 30 and the grooves 44 of the inner tube 40 are on the same axial position as the corresponding engagement structures in the form of the grooves 84 of the locking ring 8 and the ribs 94 of the receiving part 5.

As depicted in Figs. 23c, 24c, the instrument 1 is then rotated to assume the second rotational position of the outer sleeve 20 with respect to the outer tube 30, which position is indicated by the "OPERATE" indication where the second marking 72b of the outer sleeve 20 is aligned with the marking 71 of the outer tube 30. In this position, the recess 32, 42 of the outer and/or inner tube 30, 40 may abut with its circumferential edge against the inserted rod 3. This causes the outer and inner tube 30, 40 of the tube assembly 10 to rotate within the outer sleeve 20 relative to the receiving part so that the engagement structures at the instrument 1 and the engagement structures at the locking ring 8 and the receiving part 5 engage. More in detail, the rib 34 at the outer tube 30 engages the groove 84 at the locking ring 8, and the rib 94 at the receiving part 5 engages the groove 44 at the inner tube 40. For disengagement of the tube assembly 10, the outer tube 30 is rotated back into the "INTRODUCE" position of the outer sleeve 20 so that the engagement structures 34, 44 of the tubes 30, 40 can be removed along the rib-free portions 95 of the receiving part 5.

Referring now to Figs. 25a and 25b, the function of locking and unlocking of the head 13 in the head receiving portion 96 of the receiving part 5 with the instrument 1 will be explained. In Fig. 25a, the instrument 1 is in the engaged configuration of Figs. 23c, 24c where the engagement structures 34 of the outer tube 30 engage the engagement structures 84 of the locking ring 8 and the engagement structures 44 of the inner tube 40 engage the engagement structures 94 of the receiving part 5. The locking ring 8 is in an upper position in an axial direction where the head 13 is still pivotable in the head receiving portion 96 but is prevented from removal from the head receiving portion 96. The front ends 40a, 30a of the inner tube 40 and the outer tube 30 of the tube assembly 10 have their smallest distance from one another. The plunger 50 presses onto the rod that is received in the U-shaped recess 92 of the receiving part 5. The actuator assembly 100 is in the configuration "UNLOCK". In this configuration, adjustments of the angle of the receiving part 5 and the locking ring 8 relative to the bone anchoring element 4 can be performed with the instrument 1.

Fig. 25b depicts a locking position of the locking ring 8 with respect to the receiving part 5. In the locking position, the locking ring 8 is moved downward as compared to the unlocking position so that it fully clamps and locks the head 13 in the head receiving portion 96. This is achieved by moving downward the locking ring 8 with the outer tube 30 that is driven downward by the actuator assembly 100 when the actuator assembly 100 is actuated in the configuration "LOCK". As can be seen in Fig. 25b, the distance between the engagement structures of the receiving part 5 and the locking ring 8 is increased. The front end 40a of the inner tube 40 does no longer abut against the inner shoulder 35 of the outer tube 30. In the locked configuration, it is possible to pull the associated vertebra or a bone upward with the instrument 1 towards the inserted rod 3.

When the instrument 1 is engaged with the bone anchoring device 2, repeated locking and unlocking of the head 13 can be performed. To attain the locked configuration, the adjustment lever 141 of the actuator assembly 100 is rotated in the circumferential direction within the second opening area 135b of the outer sleeve 130 so that it is aligned with the indication "LOCK". Pushing the first lever arm 162 towards the handle portion 150 achieves locking of the head 13, as shown in Fig. 25b. To release the locked configuration, the first lever arm 162 of the actuator assembly 100 is no longer pushed against the handle portion 150 so that it returns to the default configuration shown in Fig. 14 by the return force generated by the spring 168. Then, the adjustment lever 141 of the actuator assembly 100 can be rotated in the circumferential direction within the second opening area 135b so that it is aligned with the indication "UNLOCK". Pushing the first lever arm 162 towards the handle portion 150 achieves unlocking of the head 13, as shown in Fig. 25a.

Finally, the instrument 1 can be removed from the bone anchoring device 2 and the fixation element 7 can be tightened to fix the locked configuration (not shown in the figures). Removing of the instrument 1 can be accomplished by the rotating outer tube 30 back into the "INTRODUCE" position of the outer sleeve 20 so that the engagement structures 34, 44 of the tubes 30, 40 can be removed along the rib-free portions 95 of the receiving part 5.

Moreover, in use of the instrument, a plurality of bone anchoring devices 2 as described above may be implanted in respective vertebrae (not shown in the figures). The rod 3 may be inserted into the receiving parts 5 of the bone anchoring devices and respective tube assemblies 10 can be connected to the bone anchoring devices. Several steps of positioning and re-positioning of the receiving parts can be performed using one single actuator assembly 100 that is selectively connectable with the different tube assemblies 10. As the actuator assembly 100 is easy to handle, the time for the adjustment steps when a plurality of bone anchoring devices have to be adjusted, may be shortened. The space needed with the instrument can be reduced. Moreover, the actuator assembly can also be removed for facilitating cleaning of the instrument.

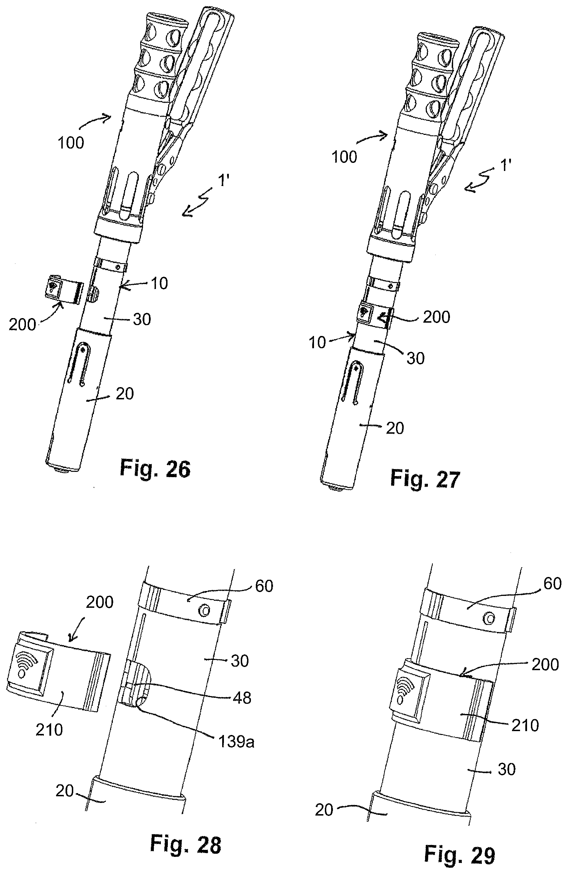

Next, a further embodiment of the instrument will be described with reference to Figs. 26 to 35b. The instrument 1' according to the further embodiment is similar to the instrument 1 of the first embodiment and in addition comprises a sensor member 200. Parts and elements of the instrument 1' that are the same as or similar to the respective parts and elements of the instrument 1 of the first embodiment described above are provided with the same reference numbers and a description thereof will be omitted.

The sensor member 200 of the instrument 1' is a separate part that is attachable to the tube assembly 10 of the instrument 1'. More in detail, the sensor member 200 can be mounted on the outer surface of the outer tube 30 at a position below the clipping part 60 by engagement with the recess 139a of the outer tube 30 and the corresponding recess 48 of the inner tube 40, as shown in Figs. 28 and 29.

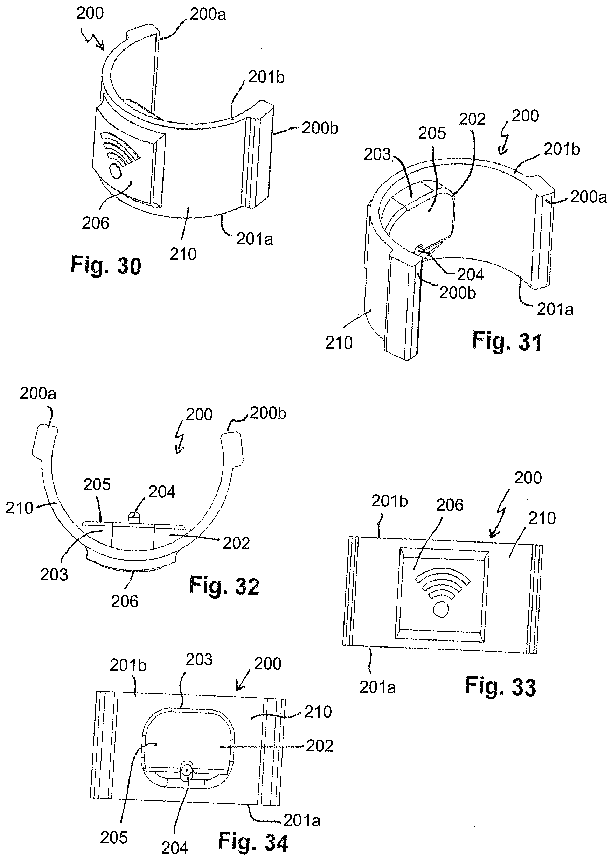

The sensor member 200 is shown in greater detail in Figs. 30 to 34. The sensor member 200 comprises a base member 210 that generally has the shape of a portion of a ring and extends between a first end 200a and a second end 200b. A circumferential extension of the base member 210 between the first and second ends may exceed half of the circumferential extension of the outer tube 30 at its outer surface. An inner surface of the base member 210 is substantially shaped complementary to the outer surface of the outer tube 30, e.g., it may be a cylindrical surface portion, so that the inner surface of the base member 210 may contact the outer surface of the outer tube in the assembled state of the sensor member 200 and the tube assembly 10. Between the first and second ends 200a, 200b an opening region is provided that allows for mounting the sensor member 200 to the outer tube. Due to the open shape of the base member 210, the base member 210 is resilient so that the first and second ends 200a, 200b can be spread apart when mounting on the outer tube 30 and snap back into their initial position when mounted thereon, which provides for a force that holds the base member 210 on the outer tube. The base member 210 may comprise thickened end portions, such as protrusions, at its first and second ends 200a, 200b, which protrusions extend away from the outer surface of the base member 210 in a radial direction.

The base member 210 extends from a lower border 201a to an upper border 201b in an axial direction, with an axial dimension of the base member 210 between the lower border 201a and the upper border 201b exceeding an axial dimension of the recess 139a of the outer tube 30 (see Fig. 28).

At the inner surface of the base member 210, and preferably in a middle portion thereof between the first and second ends 200a, 200b, a protrusion 202 is formed that protrudes from the inner surface. The protrusion 202 is shaped and sized so as to be receivable within the recess 139a or 139b of the outer tube. The protrusion 202 is bounded by a perimetral edge 203 that extends away from the inner surface of the base member 210, the perimetral edge 203 having a contour that corresponds to the contour of the recess 139a, 139b of the outer tube 30, e.g. may have a substantially elongate or oval contour with a longer axis of the contour extending in the circumferential direction of the base member 210. **In** the assembled state of the sensor member 200 and tube assembly 10, the perimetral edge 203 thus contacts the wall of the outer tube 30 that bounds the recess 139a. A rear face 205 of the protrusion 202 facing away from the inner surface of the base member may be substantially flat.

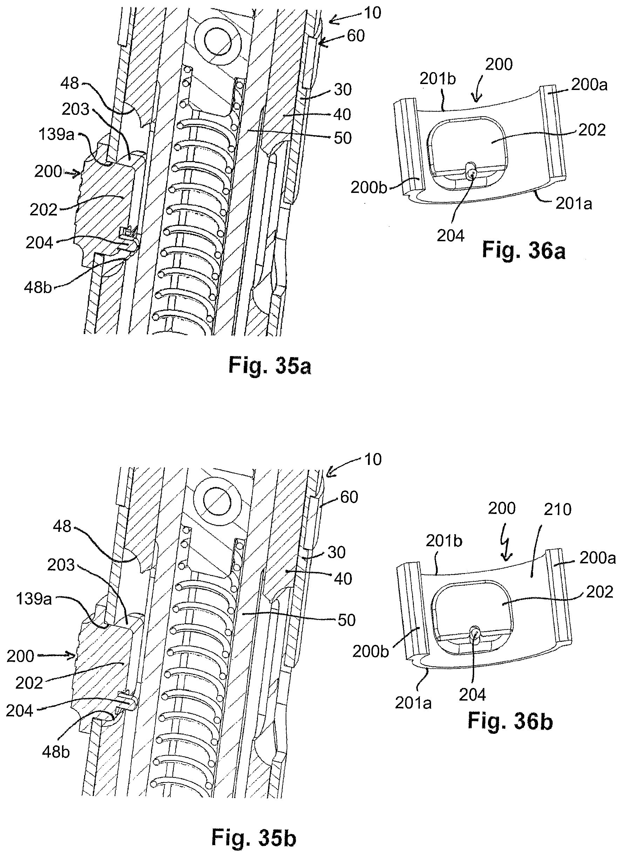

A thickness of the protrusion 202 in a direction perpendicular to the inner surface of the base member 210 may exceed a thickness of the wall of the outer tube 30 so that the protrusion 202 extends into the recess 48 of the inner tube 40 in the assembled state, as shown in Figs. 35a, 35b.

The sensor member 200 further comprises a sensor device that is not shown in the figures and may be provided within the protrusion 202. For example, the sensor device can be a sensor configured to measure a distance of a sensor element relative to a reference position, or a displacement of a sensor element, and/or can be a position sensor configured to detect the position of a sensor element. Alternatively, or in addition, the sensor device can be a pressure sensor configured to detect a pressure acting on a sensor element. The sensor element of the sensor device, such as a pin 204, extends from the sensor device to the outside of the protrusion 203. The pin 204 is movable to a certain extent relative to the protrusion 202 in the axial direction of the base member 210, i.e., towards the lower border 201a and/or upper border 201b. When the sensor member 200 is mounted on the tube assembly 10, the pin 204 protrudes into the recess 48 of the inner tube and can be contacted by the inclined lower wall portion 48b at the lower end of the recess 48, as shown in Figs. 35a, 35b. The sensor device is configured to at least detect whether or not the inclined lower wall portion 48b of the recess 48 contacts the pin 204. The sensor device may be further configured to measure a pressure that the inclined lower wall portion 48b exerts onto the pin 204 and/or to measure a relative or absolute displacement of the pin 204.

At the outer surface of the base member 210, i.e., facing away from the outer tube 30 in the assembled state of the sensor member 200 and the tube assembly 10, an indication device 206 is provided. The indication device 206 is configured to output an optical and/or audible signal in response to a measurement value of the sensor device. For example, the indication device may be configured to display two visible states, such as lights having different colours, or a light switchable on and off, wherein a first one of the visible states is assigned to a first position of the pin 204 closest to the lower border 201a of the base member 210 (see Fig. 36a) and a second one of the visible states is assigned to a second position of the pin 204 farthest away from the lower border 201a (see Fig. 36b). Alternatively or in addition, the indication device 206 may be configured to output an audible signal, e.g. a beep, when the pin 204 is in the second position farthest away from the lower border 201a (see Fig. 36b).

Referring now to Figs. 25a and 25b and Figs. 35a, 35b, 36a and 36b, the function of the sensor member 200 when locking and unlocking of the head 13 in the receiving part 5 with the instrument 1' will be explained. Fig. 35a depicts the tube assembly 10 of the instrument 1' with the sensor member 200 mounted thereon and in the unlocked configuration of the head, i.e., where the front ends 40a, 30a of the inner tube 40 and the outer tube 30 of the tube assembly 10 have their smallest distance from one another (see Fig. 25a). **In** this configuration, the lower wall portion 48b of the recess 48 of the inner tube is provided at a distance axially below the pin 204 of the sensor member 200, i.e., it does not contact the pin 204, see Fig. 35a. The pin 204 is in its first position closest to the lower border 201a of the base member 210, shown in Fig. 36a. When the instrument 1' is actuated to lock the head 13 (Fig. 25b), the inner tube 40 moves axially upwards relative to the outer tube 30 so that the lower wall portion 48b of the recess 48 of the inner tube contacts the pin 204 of the sensor member 200 and moves it towards the upper border 201b of the base member 210 into its second position, see Figs. 35b, 36b. This causes the indication device 206 to output an optical and/or audible signal to indicate the locking position of the bone anchoring device to a user.

The sensor member can thus serve to indicate the locked and/or unlocked state of the head within the receiving part and may thus provide for facilitated handling of the instrument during surgery. Moreover, if the sensor device is configured to measure a pressure that the inclined lower wall portion 48b exerts onto the pin 204 and/or a displacement of the pin 204, wear of the instrument may be detected, e.g. based on a reduced pressure value or reduced displacement determined by the sensor device in the locked state of the head.

The sensor member may be configured to transmit data to an external device, such as a computer, a navigation device, a tracker etc. Preferably, the sensor member is configured for wireless transmission of data to an external device, for example, via WiFi, Bluetooth, a radiofrequency identification (RFID) signal, etc.

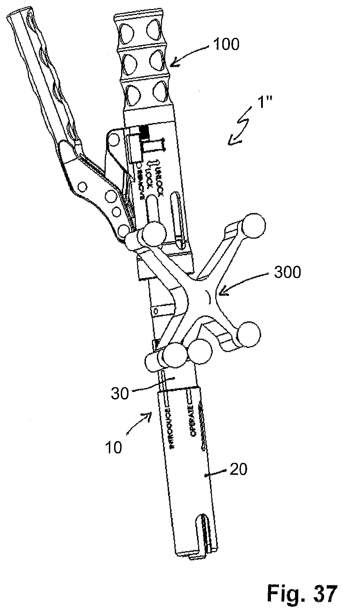

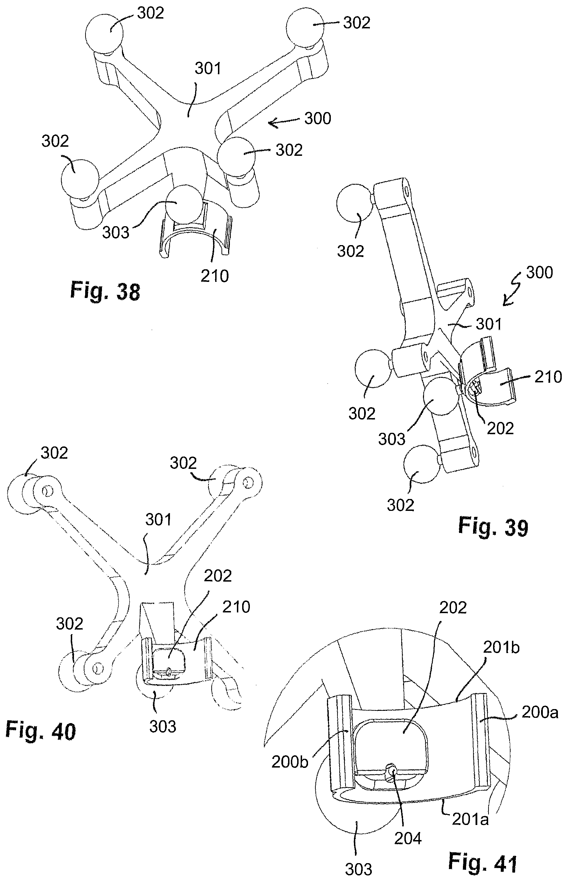

Next, a further embodiment of the instrument will be described with reference to Figs. 37 to 41. The instrument 1" according to the further embodiment is similar to the instrument 1 of the first embodiment and in addition comprises a navigation member 300. Parts and elements of the instrument 1" that are the same as or similar to the respective parts and elements of the instruments 1, 1' of the embodiments described above are provided with the same reference numbers and a description thereof will be omitted.

The navigation member 300 is a separate part that is attachable to the tube assembly 10 of the instrument 1", i.e., the navigation member 300 can be mounted on the outer surface of the outer tube 30 at a position below the clipping part 60 by engagement with the recess 139a of the outer tube 30 and the corresponding recess 48 of the inner tube 40, similar to the sensor member 200 of the instrument 1' described above. The navigation member 300 also comprises a base member 210 provided with a protrusion 202 and a movable pin 204 similar to the sensor member 200 of the instrument 1' described above. Instead of the sensor device of the sensor member 200, the navigation member 300 is provided with a navigation device in the form of a navigation star 301. The navigation star 301 is mounted on the outer surface of the base member 210 and in the present embodiment comprises four arms, each arm having a navigation sphere 302 provided at its free end. A further navigation sphere 303 is provided at the outer surface of the base member 201 and is coupled with the pin 204 such that it can assume a first position closest to the lower border 201a of the base member 210 when the pin 204 is in the first position as explained above, and the navigation sphere 303 can assume a second position farthest away from the lower border 201a when the pin 204 is in its second position as explained above.

Based on a position of the further navigation sphere 303 relative to the navigation spheres 302, the locking and unlocking configurations of the head in the receiving part can be determined, and/or a relative displacement between the inner and outer tubes of the tube assembly can be determined. Moreover, based on a position of the four navigation spheres 302, a position of the instrument can be determined.

The navigation member 300 does not need to be provided with four navigation spheres 302 fixedly connected to the base member 210, and can comprise more than four or less than four navigation spheres 302. For example, the navigation member 300 can generally comprises one or more navigation spheres 302 fixedly connected to the base member 210 and at least one further navigation sphere 303 movable relative to the base member 201 and coupled in its movement with the movement of the pin 204. A relative position or displacement of the navigation sphere 303 coupled with the movement of the pin 204 and the at least one fixed navigation sphere can be determined, for example, optically, such as by an optical beam reflected by the respective navigation spheres 302, 303.

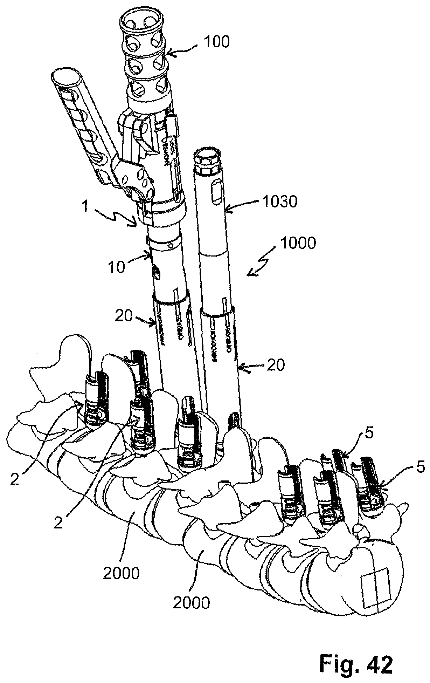

Next, a further embodiment of the instrument will be described with reference to Figs. 42 to 45. The instrument 1000 according to the further embodiment comprises a tube 1030 and an outer sleeve 20 that may be similar to the outer tube 30 and the outer sleeve 20 of the tube assembly 10 of the embodiments of the instrument described above. The tube 1030 of the instrument 1000 is attachable to the polyaxial bone anchoring device 2 described above. The instrument 1000 serves to position and reposition the receiving part 5 of the polyaxial bone anchoring device 2 relative to the bone anchoring element 4.

Turning to Fig. 42, a plurality of polyaxial bone anchoring devices 2 as described above are shown implanted in respective vertebrae 2000. The rod 3 (not shown) may be inserted in at least some of the receiving parts 5 of the polyaxial bone anchoring devices 2. The tube assembly 10 of the instrument 1 of the first embodiment described above is connected to a first one of the polyaxial bone anchoring devices 5 and the tube 1030 of the instrument 1000 is connected to a second one of the polyaxial bone anchoring devices 5. Several steps of positioning and re-positioning of the receiving parts 5 can be performed by using the instrument 1 of the first embodiment as described above, and/or the instrument 1000. For example, the instrument 1000 coupled to the second bone anchoring device 2 can be tilted and/or rotated to adapt the position of the polyaxial bone anchoring device 2 relative to the bone anchoring element 4 that is already inserted into the respective vertebrae 2000.

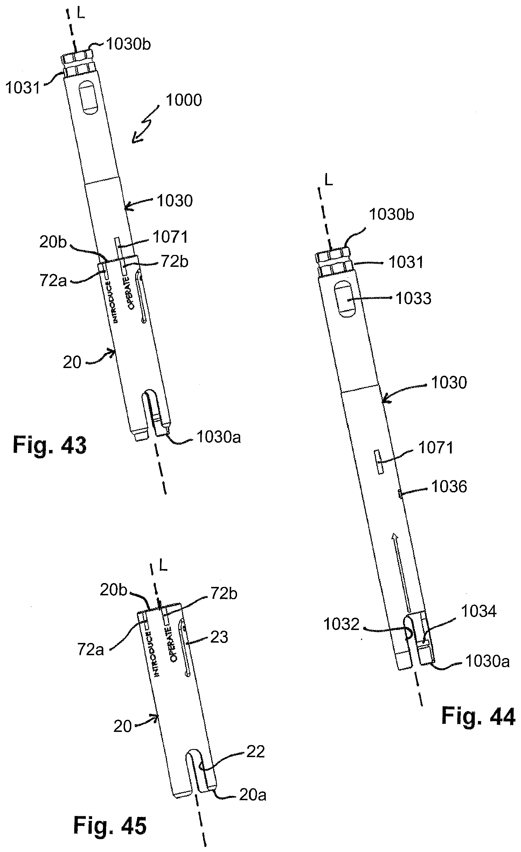

Referring additionally to Figs. 43 to 45, the tube 1030 of the instrument 1000 defines a longitudinal axis or tube axis L of the instrument 1000. In an assembled state of the instrument 1000, the outer sleeve 20 entirely encloses a lower portion of the tube 1030 in a circumferential direction thereof, the and the outer sleeve 20 is rotatable relative to the tube 1030 around the longitudinal axis L to a certain extent that permits to bring the tube 1030 into engagement with the polyaxial bone anchoring device 2.

The tube 1030 comprises a front end 1030a and a rear end 1030b opposite the front end 1030a. A recess 1032 is formed that extends from the front end 1030a of the tube 1030 to a distance thereof. The recess 1032 has a width in the circumferential direction that is larger than an upper width of the polyaxial bone anchoring device 2 and a height in the axial direction that permits the rod 3 (not shown) to extend therethrough and to be movable in an axial direction within the recess 1032. At a distance from the front end 1030a of the tube 1030 an engagement structure 1034, for example a circumferentially extending rib formed at the inner surface of the tube, is provided that is adapted to engage a corresponding engagement structure of the polyaxial bone anchoring device 2, for example the groove 84 of the locking ring 8 described above.

Adjacent to the rear end 1030b of the tube 1030 an attachment feature 1031 may be formed that serves for attachment with a further instrument or a handle portion (not shown). The attachment feature 1031 may have a polygonal shape at the outer surface of the tube 1030 to permit the further instrument or handle portion to transmit a rotational and/or tilting movement onto the instrument 1000. At a distance from the rear end 1030b an opening 1033 may be provided that facilitates cleaning or that allows to insert other instruments or parts.

At an axial distance from the attachment feature 1031 and/or the opening 1033 and further towards the front end 1030a of the tube 1030 a marking 1071 may be provided that serves to indicate a rotational position of the outer sleeve 20 with respect to the tube 1030 similar to the marking 71 of the outer tube 30 of the tube assembly 10 described above.

At an axial distance from the marking 1071 and further towards the front end 1030a of the tube 1030 two engagement features in the form of recesses 1036 are provided in the outer surface of the tube 1030, only one of which recesses 1036 can be seen in the figures. The recesses 1036 are spaced apart in a circumferential direction of the tube 1030 and serve for receiving corresponding engagement features, such as engagement latches, of the outer sleeve 20 in the respective rotational positions of the outer sleeve 20, similar to the recesses 36 of the outer tube 30 of the tube assembly 10 described above.

The outer sleeve 20 is similar to the outer sleeve of the instrument described above. As shown in Fig. 45, the outer sleeve 20 comprises a front end 20a rear end 20b and an internal channel extending from the front end 20a to the rear end 20b, wherein the front portion of the tube 1030 can be received in the internal channel of the outer sleeve 20. The recess 22 formed in the front region of the outer sleeve 20 adjacent its front end 20a has a width in the circumferential direction that substantially corresponds to or is slightly larger than a diameter of the rod 3 and is smaller than the width of the recesses 1032 of the tube 1030. An axial length of the outer sleeve 20 between the front end 20a and the rear end 20b may be shorter than an axial length of the tube 1030 and may be such that the rear end 20b is substantially located in a region of the marking 1071 at the tube 1030 when the outer sleeve 20 is assembled with the tube 1030, as shown in Fig. 43.

The latching tab 23 of the outer sleeve 20 is configured to selectively engage one of the two recesses 1036 provided at the outer surface of the tube 1030 to attach the outer sleeve 20 to the tube 1030 at the first rotational position and the second rotational position, respectively, as described above for the outer sleeve 20 and the outer tube 30 of the tube assembly. **In** the first rotational position of the outer sleeve 20 indicated by the "INTRODUCE" indication where the first marking 72a of the outer sleeve 20 is aligned with the marking 1071 of the tube 1030, the tube 1030 can be placed onto the receiving part 5 of the polyaxial bone anchoring device 2 and is removable therefrom. In the second rotational position of the outer sleeve 20, indicated by the "OPERATE" indication where the second marking 72b of the outer sleeve 20 is aligned with the marking 1071 of the tube 1030, the engagement structure 1034 of the tube 1030 engages the corresponding engagement structure of the polyaxial bone anchoring device 2 and is not removable therefrom.

In use, the instrument 1000 is first attached to the polyaxial bone anchoring device 2 with the bone anchoring element 4 already implanted in a vertebra 2000 or other bone. The rod 3 may be inserted into the receiving part. The instrument 1000 is in the first rotational position of the outer sleeve 20 with respect to the tube 1030, which position is indicated by the "INTRODUCE" indication where the first marking 72a of the outer sleeve 20 is aligned with the marking 1071 of the tube 1030. In this configuration, the instrument 1000 is placed onto the receiving part 5 of the bone anchoring device 2 and moved downward in such an orientation that the recess 22 of the outer sleeve 20 is aligned with the substantially U-shaped recess 92 of the receiving part 5 that forms a channel for the rod 3, as described above for the instrument 1. As described above for the instrument 1, in this configuration the engagement structure 1034 of the tube 1030 is circumferentially aligned with the rib-free outer surface portion 95 of the receiving part 5. The outer sleeve 20 with its recess 22 assists in placement of the instrument 1000 onto the receiving part in the correct orientation and may therefore serve as an alignment member that assists in attaching the tube 1030 to the bone anchoring device 2.

Next, when the engagement structure 1034 of the tube 1030 is at the same axial position as the corresponding engagement structure of the bone anchoring device, the instrument 1000 is rotated to assume the second rotational position of the outer sleeve 20 with respect to the tube 1030. This position is indicated by the "OPERATE" indication where the second marking 72b of the outer sleeve 20 is aligned with the marking 1071 of the tube 1030. In this position, the recess 1032 of the tube 1030 may abut with its circumferential edge against the inserted rod 3. The tube 1030 is thus rotated within the outer sleeve 20 relative to the receiving part so that the engagement structure at the instrument 1000, i.e., the rib 1034 of the tube 1030, and the engagement structure at the bone anchoring device, i.e., the groove 84 of the locking ring 8, engage. In the engaged configuration of the instrument 1000 and the bone anchoring device 2, the instrument 1000 can be tilted and/or rotated so as to adapt the position of the receiving part relative to the bone anchoring element.

For disengagement of the instrument 1000, the tube 1030 is rotated back into the "INTRODUCE" position of the outer sleeve 20 so that the engagement structure 1034 of the tube 1030 can be removed along the rib-free portions 95 of the receiving part 5, as explained above for the instrument 1.

It shall be noted that one or several tubes 1030 can be used with corresponding outer sleeves 20, respectively, in addition or alternatively to the tube assembly 1.

Various modifications of the above embodiments of the instrument and bone anchoring device can be contemplated. For example, the arrangement, number, configuration and shape of the protrusions that form the driving and the driven portions may be different. Although the instrument is shown together with a polyaxial bone anchoring device of the bottom loading type with an outer locking ring, the instrument is not limited to be used only with such a device. For example, the instrument can be used with any type of polyaxial bone anchoring device in which a clamping means is axially displaceable to clamp an inserted head, or with a monoaxial or monoplanar bone anchoring device.

## Claims

1. An instrument for use with a bone anchoring device (2), the bone anchoring device (2) comprising a bone anchoring element (4) and a receiving part (5) for coupling a rod (3) to the bone anchoring element (4), wherein the instrument (1, 1', 1") is an instrument for locking and unlocking a head (13) of the bone anchor in the receiving part (5) of the bone anchoring device (2), and the instrument comprises
a tube assembly (10) comprising at least a first tube (40) and a second tube (30) that define a longitudinal axis (L), the first and second tubes being configured to engage the bone anchoring device (2) and being movable relative to one another between a first axial position associated with an unlocking configuration in which the head (13) is unlocked in the receiving part (5), and a second axial position associated with a locking configuration in which the head (13) is locked in the receiving part (5), and
at least one alignment member (20, 50) that is configured to assist in attaching the tube assembly (10) to the bone anchoring device (2) by guiding the tube assembly relative to the receiving part of the bone anchoring device.

2. The instrument of claim 1, wherein the alignment member (20, 50) is a member separate from the tube (10, 30, 40, 1030) and preferably attachable to the tube.

3. The instrument of claim 1 or 2, wherein the alignment member is an outer alignment member, preferably in the form of an outer sleeve (20), that at least partially, preferably entirely, encloses the tube (10, 30, 40, 1030) in a circumferential direction thereof.

4. The instrument of claim 3, wherein the alignment member (20) is movable relative to the tube (10, 30, 40, 1030) between a first rotational position in which the tube (10, 30, 40, 1030) is attachable to and detachable from the bone anchoring device (2), and a second rotational position in which the tube (10, 30, 40, 1030) is configured to engage the bone anchoring device (2) and is prevented from being removed therefrom.

5. The instrument of claim 3 or 4, wherein the outer alignment member (20) has a first end (20b) and a second end (20a) and comprises a recess (22) open to the second end (20a), the recess (22) being oriented and formed to align with a substantially U-shaped recess (92) provided at the receiving part (5) for receiving a rod (3),
preferably wherein the tube (10, 30, 40, 1030) has a first end (30b, 40b, 1030b) and a second end (30a, 30b, 1030b) and comprises a recess (32, 42, 1032) open to the second end, and wherein a width of the recess (22) of the alignment member (20) in the circumferential direction is smaller than a width of the recesses (32, 42, 1032) of the tube (10, 30, 40, 1030) such that the recess (22) of the alignment member and the recess (32, 42, 1032) of the tube overlap in both the first rotational position and the second rotational position to permit an inserted rod (3) to pass therethrough.

6. The instrument of one of claims 1 to 5, wherein the instrument further comprises an actuator assembly (100) comprising an actuating mechanism configured to move the first and second tubes of the tube assembly (10) from the first axial position to the second axial position, and vice versa,
wherein preferably the actuator assembly (100) is a separate part that is connectable to and removable from the tube assembly (10).

7. The instrument of one of claims 1 to 6, wherein the alignment member is an inner alignment member, preferably in the form of a plunger (50), arranged at least partially, preferably entirely, within the at least one tube (10, 30, 40, 1030).

8. The instrument of claim 7, wherein the inner alignment member (50) is configured to enter a coaxial bore (91) of the receiving part (5) of the bone anchoring device when the at least one tube is attached to the bone anchoring device, preferably wherein the inner alignment member (50) is configured to press onto a rod (3) inserted into the receiving part.

9. The instrument of one of claims 6 to 8, wherein the actuator assembly (100) is configured to assume a first configuration wherein the tube assembly (10) is configured to assume the first axial position when the actuating mechanism is actuated, and a second configuration wherein the tube assembly (10) is configured to assume the second axial position when the actuating mechanism is actuated, and a third configuration in which the actuator assembly (100) is connectable to and/or removable from the tube assembly (10).

10. The instrument of claim 9, wherein the actuator assembly (100) comprises an adjustment member (140) configured to rotate in the circumferential direction to selectively place the actuator assembly (100) in the first or second or third configuration, wherein preferably the adjustment member (140) is secured against movement into a position associated with the third configuration.

11. The instrument of claim 10, wherein the actuator assembly (100) comprises a handle portion (150) connectable to an outer sleeve (130) of the actuator assembly and the adjustment member (140) is only movable into a position associated with the third configuration if the handle portion (150) is disconnected, in particular unthreaded, from the outer sleeve (130) to a certain extent.

12. The instrument of one of claims 9 to 11, wherein the actuator assembly (100) comprises at least two driving portions (122, 142) that are configured to be displaceable relative to one another parallel to the longitudinal axis in response to an actuation of the actuating mechanism and wherein the tube assembly (10) comprises at least two driven portions (45a, 45b) that are configured to be driven by the driving portions (122, 142) and wherein the at least two driven portions (45a, 45b) are circumferentially spaced apart from one another corresponding to the first and second configurations of the actuator assembly (100), preferably wherein by the circumferentially spaced apart driven portions (45a, 45b) a gap (G) is provided that permits the driving portions (122, 142) to enter the tube assembly (10) and that defines the third configuration of the actuator assembly (100).

13. The instrument of one of claims 1 to 12, further comprising a sensor member (200) and/or a navigation member (300) configured to detect at least one of the unlocking configuration and the locking configuration of the head (13) in the receiving part (5) or the first axial position and the second axial position of the first and second tubes.

14. The instrument of one of claims 6 to 13, wherein the actuator assembly (100) is configured to assume at least a third configuration in which the actuator assembly (100) is connectable to and/or removable from the tube assembly (10), and a securing mechanism preventing the actuator assembly (100) from assuming the third configuration,
preferably wherein the securing mechanism can be released by unscrewing a handle portion (150) of the actuator assembly.

15. A system of an instrument according to one of claims 1 to 14 and a bone anchoring device, wherein the bone anchoring device (2) comprises a bone anchoring element (4) for anchoring in a bone and a receiving part (5) for receiving a head (13) of the bone anchoring element and wherein a clamping device (8) is provided that acts upon the head (13) in the receiving part to lock the head (13) in the receiving part, and wherein the bone anchoring device (2) comprises an engagement structure (84, 94) configured to be engaged by an engagement structure (34, 44, 1034) at the tube (10, 30, 40, 1030),
wherein preferably the receiving part (5) and the clamping device (8) each comprise an engagement structure (84, 94) configured to be engaged by an engagement structure (34, 44) at the first tube (40) and the second tube (30), respectively.

## Patentansprüche

1. Instrument zur Verwendung mit einer Knochenverankerungsvorrichtung (2), wobei die Knochenverankerungsvorrichtung (2) ein Knochenverankerungselement (4) und ein Aufnahmeteil (5) zum Koppeln eines Stabes (3) mit dem Knochenverankerungselement (4) umfasst, wobei das Instrument (1, 1', 1'') ein Instrument zum Verriegeln und Entriegeln eines Kopfes (13) des Knochenankers im Aufnahmeteil (5) der Knochenverankerungsvorrichtung (2) ist und das Instrument umfasst:
eine Rohranordnung (10), die mindestens ein erstes Rohr (40) und ein zweites Rohr (30) umfasst, welche eine Längsachse (L) definieren, wobei das erste und das zweite Rohr dazu ausgebildet sind, mit der Knochenverankerungsvorrichtung (2) in Eingriff zu sein, und relativ zueinander bewegbar sind zwischen einer ersten axialen Position, die einer Entriegelungskonfiguration zugeordnet ist, in der der Kopf (13) im Aufnahmeteil (5) entriegelt ist, und einer zweiten axialen Position, die einer Verriegelungskonfiguration zugeordnet ist, in der der Kopf (13) im Aufnahmeteil (5) verriegelt ist, und
mindestens ein Ausrichtungselement (20, 50), das dazu ausgebildet ist, das Anbringen der Rohranordnung (10) an der Knochenverankerungsvorrichtung (2) zu unterstützen, indem es die Rohranordnung relativ zum Aufnahmeteil der Knochenverankerungsvorrichtung führt.

2. Instrument nach Anspruch 1, wobei das Ausrichtungselement (20, 50) ein vom Rohr (10, 30, 40, 1030) separates Element ist und vorzugsweise am Rohr befestigbar ist.

3. Instrument nach Anspruch 1 oder 2, wobei das Ausrichtungselement ein äußeres Ausrichtungselement ist, vorzugsweise in Form einer Außenhülse (20), das das Rohr (10, 30, 40, 1030) in dessen Umfangsrichtung zumindest teilweise, vorzugsweise vollständig, umschließt.

4. Instrument nach Anspruch 3, wobei das Ausrichtungselement (20) relativ zum Rohr (10, 30, 40, 1030) bewegbar ist zwischen einer ersten Drehposition, in der das Rohr (10, 30, 40, 1030) an der Knochenverankerungsvorrichtung (2) befestigbar und von dieser lösbar ist, und einer zweiten Drehposition, in der das Rohr (10, 30, 40, 1030) dazu ausgebildet ist, mit der Knochenverankerungsvorrichtung (2) in Eingriff zu sein und ein Lösen von dieser verhindert ist.

5. Instrument nach Anspruch 3 oder 4, wobei das äußere Ausrichtungselement (20) ein erstes Ende (20b) und ein zweites Ende (20a) aufweist und eine zum zweiten Ende (20a) hin offene Ausnehmung (22) umfasst, wobei die Ausnehmung (22) so orientiert und ausgebildet ist, um mit einer im Wesentlichen U-förmigen Ausnehmung (92) ausgerichtet zu sein, die am Aufnahmeteil (5) zur Aufnahme eines Stabes (3) vorgesehen ist,
wobei vorzugsweise das Rohr (10, 30, 40, 1030) ein erstes Ende (30b, 40b, 1030b) und ein zweites Ende (30a, 30b, 1030b) aufweist und eine zum zweiten Ende hin offene Ausnehmung (32, 42, 1032) umfasst, und wobei eine Breite der Ausnehmung (22) des Ausrichtungselements (20) in Umfangsrichtung kleiner ist als eine Breite der Ausnehmungen (32, 42, 1032) des Rohrs (10, 30, 40, 1030), so dass sich die Ausnehmung (22) des Ausrichtungselements und die Ausnehmung (32, 42, 1032) des Rohrs sowohl in der ersten Drehposition als auch in der zweiten Drehposition überlappen, um zu erlauben, dass sich ein eingesetzter Stab (3) hindurch erstreckt.

6. Instrument nach einem der Ansprüche 1 bis 5, wobei das Instrument weiter eine Betätigungsanordnung (100) umfasst, die einen Betätigungsmechanismus umfasst, der dazu ausgebildet ist, das erste und das zweite Rohr der Rohranordnung (10) von der ersten axialen Position in die zweite axiale Position und umgekehrt zu bewegen,
wobei die Betätigungsanordnung (100) vorzugsweise ein separates Teil ist, das mit der Rohranordnung (10) verbindbar und von dieser lösbar ist.

7. Instrument nach einem der Ansprüche 1 bis 6, wobei das Ausrichtungselement ein inneres Ausrichtungselement ist, vorzugsweise in Form eines Stößels (50), das zumindest teilweise, vorzugsweise vollständig, innerhalb des mindestens einen Rohrs (10, 30, 40, 1030) angeordnet ist.

8. Instrument nach Anspruch 7, wobei das innere Ausrichtungselement (50) dazu ausgebildet ist, in eine koaxiale Bohrung (91) des Aufnahmeteils (5) der Knochenverankerungsvorrichtung einzutreten, wenn das mindestens eine Rohr an der Knochenverankerungsvorrichtung angebracht ist, wobei vorzugsweise das innere Ausrichtungselement (50) dazu ausgebildet ist, auf einen in das Aufnahmeteil eingeführten Stab (3) zu drücken.

9. Instrument nach einem der Ansprüche 6 bis 8, wobei die Betätigungsanordnung (100) dazu ausgebildet ist, eine erste Konfiguration einzunehmen, wobei die Rohranordnung (10) dazu ausgebildet ist die erste axiale Position einzunehmen, wenn der Betätigungsmechanismus betätigt wird, sowie eine zweite Konfiguration, wobei die Rohranordnung (10) dazu ausgebildet ist die zweite axiale Position einzunehmen, wenn der Betätigungsmechanismus betätigt wird, und eine dritte Konfiguration, in der die Betätigungsanordnung (100) mit der Rohranordnung (10) verbindbar und/oder von dieser lösbar ist.

10. Instrument nach Anspruch 9, wobei die Betätigungsanordnung (100) ein Einstellelement (140) umfasst, das dazu ausgebildet ist, sich in der Umfangsrichtung zu drehen, um die Betätigungsanordnung (100) wahlweise in die erste, zweite oder dritte Konfiguration zu bringen, wobei das Einstellelement (140) vorzugsweise gegen eine Bewegung in eine der dritten Konfiguration zugeordnete Position gesichert ist.

11. Instrument nach Anspruch 10, wobei die Betätigungsanordnung (100) einen Griffabschnitt (150) umfasst, der mit einer Außenhülse (130) der Betätigungsanordnung verbindbar ist, und das Einstellelement (140) nur dann in eine der dritten Konfiguration zugeordnete Position bewegbar ist, wenn der Griffabschnitt (150) zu einem gewissen Ausmaß von der Außenhülse (130) getrennt, insbesondere abgeschraubt, ist.

12. Instrument nach einem der Ansprüche 9 bis 11, wobei die Betätigungsanordnung (100) mindestens zwei Antriebsabschnitte (122, 142) umfasst, die so ausgebildet sind, dass sie als Reaktion auf eine Betätigung des Betätigungsmechanismus relativ zueinander parallel zur Längsachse verschiebbar sind, und wobei die Rohranordnung (10) mindestens zwei angetriebene Abschnitte (45a, 45b) umfasst, die so ausgebildet sind, dass sie von den Antriebsabschnitten (122, 142) antreibbar sind, und wobei die mindestens zwei angetriebenen Abschnitte (45a, 45b) entsprechend der ersten und zweiten Konfiguration der Betätigungsanordnung (100) in Umfangsrichtung voneinander beabstandet sind, vorzugsweise wobei durch die in Umfangsrichtung voneinander beabstandeten angetriebenen Abschnitte (45a, 45b) ein Spalt (G) vorgesehen ist, der es den Antriebsabschnitten (122, 142) ermöglicht, in die Rohranordnung (10) einzutreten, und der die dritte Konfiguration der Betätigungsanordnung (100) definiert.

13. Instrument nach einem der Ansprüche 1 bis 12, weiter umfassend ein Sensorelement (200) und/oder ein Navigationselement (300), das zum Erfassen zumindest der Entriegelungskonfiguration und der Verriegelungskonfiguration des Kopfes (13) im Aufnahmeteil (5), oder der ersten axialen Position und der zweiten axialen Position des ersten und des zweiten Rohrs ausgebildet ist.

14. Instrument nach einem der Ansprüche 6 bis 13, wobei die Betätigungsanordnung (100) dazu ausgebildet ist, mindestens eine dritte Konfiguration einzunehmen, in der die Betätigungsanordnung (100) mit der Rohranordnung (10) verbindbar und/oder von dieser lösbar ist, und ein Sicherungsmechanismus verhindert, dass die Betätigungsanordnung (100) die dritte Konfiguration einnimmt,
wobei vorzugsweise der Sicherungsmechanismus durch Abschrauben eines Griffabschnitts (150) der Betätigungsanordnung freigegeben werden kann.

15. System aus einem Instrument gemäß einem der Ansprüche 1 bis 14 und einer Knochenverankerungsvorrichtung, wobei die Knochenverankerungsvorrichtung (2) ein Knochenverankerungselement (4) zur Verankerung in einem Knochen und ein Aufnahmeteil (5) zur Aufnahme eines Kopfes (13) des Knochenverankerungselements umfasst und wobei eine Klemmvorrichtung (8) vorgesehen ist, die auf den Kopf (13) im Aufnahmeteil wirkt, um den Kopf (13) im Aufnahmeteil zu verriegeln, und wobei die Knochenverankerungsvorrichtung (2) eine Eingriffsstruktur (84, 94) umfasst, die dazu ausgebildet ist, mit einer Eingriffsstruktur (34, 44, 1034) am Rohr (10, 30, 40, 1030) in Eingriff zu sein,
wobei vorzugsweise das Aufnahmeteil (5) und die Klemmvorrichtung (8) jeweils eine Eingriffsstruktur (84, 94) umfassen, die dazu ausgebildet ist, mit einer Eingriffsstruktur (34, 44) am ersten Rohr (40) bzw. am zweiten Rohr (30) in Eingriff zu sein.

## Revendications

1. Instrument destiné à être utilisé avec un dispositif d'ancrage osseux (2), le dispositif d'ancrage osseux (2) comprenant un élément d'ancrage osseux (4) et une partie de réception (5) pour coupler une tige (3) à l'élément d'ancrage osseux (4), dans lequel l'instrument (1, 1', 1") est un instrument pour verrouiller et déverrouiller une tête (13) de l'ancrage osseux dans la partie de réception (5) du dispositif d'ancrage osseux (2), et l'instrument comprend
un ensemble de tube (10) comprenant au moins un premier tube (40) et un second tube (30) qui définissent un axe longitudinal (L), les premier et second tubes étant configurés pour venir en prise avec le dispositif d'ancrage osseux (2) et étant mobiles l'un par rapport à l'autre entre une première position axiale associée à une configuration de déverrouillage dans laquelle la tête (13) est déverrouillée dans la partie de réception (5), et une seconde position axiale associée à une configuration de verrouillage dans laquelle la tête (13) est verrouillée dans la partie de réception (5), et
au moins un élément d'alignement (20, 50) qui est configuré pour aider à fixer l'ensemble de tube (10) au dispositif d'ancrage osseux (2) en guidant l'ensemble de tube par rapport à la partie de réception du dispositif d'ancrage osseux.

2. Instrument selon la revendication 1, dans lequel l'élément d'alignement (20, 50) est un élément séparé du tube (10, 30, 40, 1030) et de préférence pouvant être fixé au tube.

3. Instrument selon la revendication 1 ou 2, dans lequel l'élément d'alignement est un élément d'alignement externe, de préférence sous la forme d'un manchon externe (20), qui entoure au moins partiellement, de préférence entièrement, le tube (10, 30, 40, 1030) dans une direction circonférentielle de celui-ci.

4. Instrument selon la revendication 3, dans lequel l'élément d'alignement (20) est mobile par rapport au tube (10, 30, 40, 1030) entre une première position de rotation dans laquelle le tube (10, 30, 40, 1030) peut être fixé au et détaché du dispositif d'ancrage osseux (2), et une seconde position de rotation dans laquelle le tube (10, 30, 40, 1030) est configuré pour venir en prise avec le dispositif d'ancrage osseux (2) et ne peut pas en être retiré.

5. Instrument selon la revendication 3 ou 4, dans lequel l'élément d'alignement externe (20) a une première extrémité (20b) et une seconde extrémité (20a) et comprend un évidement (22) ouvert vers la seconde extrémité (20a), l'évidement (22) étant orienté et formé pour s'aligner avec un évidement sensiblement en forme de U (92) prévu au niveau de la partie de réception (5) pour recevoir une tige (3),
de préférence dans lequel le tube (10, 30, 40, 1030) a une première extrémité (30b, 40b, 1030b) et une seconde extrémité (30a, 30b, 1030b) et comprend un évidement (32, 42, 1032) ouvert vers la seconde extrémité, et dans lequel une largeur de l'évidement (22) de l'élément d'alignement (20) dans la direction circonférentielle est inférieure à une largeur des évidements (32, 42, 1032) du tube (10, 30, 40, 1030) de sorte que l'évidement (22) de l'élément d'alignement et l'évidement (32, 42, 1032) du tube se chevauchent à la fois dans la première position de rotation et la seconde position de rotation pour permettre à une tige insérée (3) de passer à travers ceux-ci.

6. Instrument selon l'une des revendications 1 à 5, dans lequel l'instrument comprend en outre un ensemble actionneur (100) comprenant un mécanisme d'actionnement configuré pour déplacer les premier et second tubes de l'ensemble tube (10) de la première position axiale à la seconde position axiale, et vice versa,
dans lequel de préférence l'ensemble actionneur (100) est une partie séparée qui peut être reliée à et retirée de l'ensemble de tube (10).

7. Instrument selon l'une des revendications 1 à 6, dans lequel l'élément d'alignement est un élément d'alignement interne, de préférence sous la forme d'un plongeur (50), agencé au moins partiellement, de préférence entièrement, à l'intérieur de l'au moins un tube (10, 30, 40, 1030).

8. Instrument selon la revendication 7, dans lequel l'élément d'alignement interne (50) est configuré pour pénétrer dans un alésage coaxial (91) de la partie de réception (5) du dispositif d'ancrage osseux lorsque l'au moins un tube est fixé au dispositif d'ancrage osseux, de préférence dans lequel l'élément d'alignement interne (50) est configuré pour appuyer sur une tige (3) insérée dans la partie de réception.

9. Instrument selon l'une des revendications 6 à 8, dans lequel l'ensemble actionneur (100) est configuré pour adopter une première configuration dans laquelle l'ensemble tube (10) est configuré pour adopter la première position axiale lorsque le mécanisme d'actionnement est actionné, et une deuxième configuration dans laquelle l'ensemble tube (10) est configuré pour adopter la seconde position axiale lorsque le mécanisme d'actionnement est actionné, et une troisième configuration dans laquelle l'ensemble actionneur (100) peut être relié à et/ou retiré de l'ensemble tube (10).

10. Instrument selon la revendication 9, dans lequel l'ensemble actionneur (100) comprend un élément d'ajustement (140) configuré pour tourner dans la direction circonférentielle pour placer sélectivement l'ensemble actionneur (100) dans la première ou la deuxième ou la troisième configuration, dans lequel de préférence l'élément d'ajustement (140) est fixé contre un mouvement dans une position associée à la troisième configuration.

11. Instrument selon la revendication 10, dans lequel l'ensemble actionneur (100) comprend une partie de poignée (150) pouvant être reliée à un manchon externe (130) de l'ensemble actionneur et l'élément d'ajustement (140) est seulement mobile dans une position associée à la troisième configuration si la partie de poignée (150) est déconnectée, en particulier retirée, du manchon externe (130) dans une certaine mesure.

12. Instrument selon l'une des revendications 9 à 11, dans lequel l'ensemble actionneur (100) comprend au moins deux parties d'entraînement (122, 142) qui sont configurées pour être déplaçables l'une par rapport à l'autre parallèlement à l'axe longitudinal en réponse à un actionnement du mécanisme d'actionnement et dans lequel l'ensemble tube (10) comprend au moins deux parties entraînées (45a, 45b) qui sont configurées pour être entraînées par les parties d'entraînement (122, 142) et dans lequel les au moins deux parties entraînées (45a, 45b) sont espacées circonférentiellement l'une de l'autre correspondant aux première et deuxième configurations de l'ensemble actionneur (100), de préférence dans lequel par les parties entraînées espacées circonférentiellement (45a, 45b), un espace (G) est prévu qui permet aux parties d'entraînement (122, 142) de pénétrer dans l'ensemble tube (10) et qui définit la troisième configuration de l'ensemble actionneur (100).

13. Instrument selon l'une des revendications 1 à 12, comprenant en outre un élément de capteur (200) et/ou un élément de navigation (300) configuré pour détecter au moins l'une de la configuration de déverrouillage et de la configuration de verrouillage de la tête (13) dans la partie de réception (5) ou la première position axiale et la seconde position axiale des premier et second tubes.

14. Instrument selon l'une des revendications 6 à 13, dans lequel l'ensemble actionneur (100) est configuré pour adopter au moins une troisième configuration dans laquelle l'ensemble actionneur (100) peut être relié à et/ou retiré de l'ensemble tube (10), et un mécanisme de fixation empêchant l'ensemble actionneur (100) d'adopter la troisième configuration,
de préférence dans lequel le mécanisme de fixation peut être libéré en dévissant une partie de poignée (150) de l'ensemble actionneur.

15. Système d'un instrument selon l'une des revendications 1 à 14 et d'un dispositif d'ancrage osseux, dans lequel le dispositif d'ancrage osseux (2) comprend un élément d'ancrage osseux (4) pour l'ancrage dans un os et une partie de réception (5) pour recevoir une tête (13) de l'élément d'ancrage osseux et dans lequel un dispositif de serrage (8) est prévu qui agit sur la tête (13) dans la partie de réception pour verrouiller la tête (13) dans la partie de réception, et dans lequel le dispositif d'ancrage osseux (2) comprend une structure de mise en prise (84, 94) configurée pour être mise en prise par une structure de mise en prise (34, 44, 1034) au niveau du tube (10, 30, 40, 1030),
dans lequel de préférence la partie de réception (5) et le dispositif de serrage (8) comprennent chacun une structure de mise en prise (84, 94) configurée pour être mise en prise par une structure de mise en prise (34, 44) au niveau du premier tube (40) et du second tube (30), respectivement.
